# EUROPÄISCHE PATENTSCHRIFT

(11) **EP 3 876 840 B1**
(45) Veröffentlichungstag und Bekanntmachung des Hinweises auf die Patenterteilung: **03.04.2024**
(21) Anmeldenummer: 19729222.0
(22) Anmeldetag: 03.06.2019
(51) Int. Cl.: A61B 5/145, G16H 10/40, A61B 5/01, G16H 50/20

(54) **VORRICHTUNG UND VERFAHREN ZUM ANALYSIEREN EINES STOFFS**
APPARATUS AND METHOD FOR ANALYZING A SUBSTANCE
DISPOSITIF ET PROCÉDÉ POUR L'ANALYSE D'UNE SUBSTANCE

(30) Priorität: 08.11.2018 WO PCT/EP2018/080665
(43) Veröffentlichungstag der Anmeldung: 15.09.2021
(73) Patentinhaber: DiaMonTech AG, 10245 Berlin (DE)
(72) Erfinder: SCHRIEK, Uwe, 10627 Berlin (DE); NOELL, Wilfried, 2000 Neuchatel (CH); LUBINSKI, Thorsten, 10245 Berlin (DE)
(74) Vertreter: Lucke, Andreas
(86) Internationale Anmeldenummer: PCT/EP2019/064356
(87) Internationale Veröffentlichungsnummer: WO 2020/094265

(56) Entgegenhaltungen:
- WO-A1-2017/097824
- US-A1- 2014 114 187
- US-A1- 2017 042 428
- BAUER-MARSCHALLINGER J ET AL: "Photoacoustic scanning macroscopy with interferometric ultrasound detection based on a fiber optic ring array", PROGRESS IN BIOMEDICAL OPTICS AND IMAGING, SPIE - INTERNATIONAL SOCIETY FOR OPTICAL ENGINEERING, BELLINGHAM, WA, US, Bd. 10494, 19. Februar 2018 (2018-02-19), Seiten 104945V-104945V, XP060102166, ISSN: 1605-7422, DOI: 10.1117/12.2289639 ISBN: 978-1-5106-0027-0

## Beschreibung

Das vorliegende Schutzrecht bezieht sich auf eine Vorrichtung und ein Verfahren zum Analysieren eines Stoffs. Diese können zum Beispiel zur Analyse von tierischem oder menschlichem Gewebe, Flüssigkeiten, insbesondere Körperflüssigkeiten und in einer Ausführungsform zur Messung von Glukose bzw. Blutzucker, genutzt werden.

Bekannte Verfahren zum Analysieren eines Stoffs, insbesondere zum Messen von Blutzucker, sind beispielsweise in den folgenden Druckschriften beschrieben:
1. Guo et al.: "Noninvasive glucose detection in human skin using wavelength modulated differential laser photothermal radiometry", Biomedical Optics Express, Vol, 3, 2012, No. 11,
2. Uemura et al.: "Non-invasive blood glucose measurement by Fourier transform infrared spectroscopic analysis through the mucous membrane of the lip: application of a chalcogenide optical fiber system", Front Med Biol Eng. 1999; 9(2): 137-153,
3. Farahi et al.: "Pump probe photothermal spectroscopy using quantum cascade lasers", J. Phys. D. Appl. Phys. 45 (2012) und
4. M. Fujinami et al.: "Highly sensitive detection of molecules at the liquid/liquid interface using total internal reflection-optical beam deflection based on photothermal spectroscopy", Rev. Sci. Instrum., Vol. 74, Number 1 (2003).
5. von Lilienfeld-Toal, H. Weidenmüller, M. Xhelaj, A. Mäntele, W. A Novel Approach to Non- Invasive Glucose Measurement by Mid-Infrared Spectroscopy: The Combination of Quantum Cascade Lasers (QCL) and Photoacoustic Detection Vibrational Spectroscopy, 38:209-215, 2005.
6. Pleitez, M. von Lilienfeld-Toal, H. Mäntele W. Infrared spectroscopic analysis of human interstitial fluid in vitro and in vivo using FT-IR spectroscopy and pulsed quantum cascade lasers (QCL): Establishing a new approach to non invasive glucose measurement Spectrochimica Acta. Part A, Molecular and biomolecular spectroscopy, 85:61-65, 2012
7. Pleitez, M. et al. In Vivo Noninvasive Monitoring of Glucose Concentration in Human Epidermis by Mid-Infrared Pulsed Photoacoustic Spectroscopy Analytical Chemistry, 85:1013-1020, 2013.
8. Pleitez, M. Lieblein, T. Bauer, A. Hertzberg, O. von Lilienfeld-Toal, H. Mäntele, W. Windowless ultrasound photoacoustic cell for in vivo mid-IR spectroscopy of human epidermis: Low interference by changes of air pressure, temperature, and humidity caused by skin contact opens the possibility for a non-invasive monitoring of glucose in the interstitial fluid Review of Scientific Instruments 84, 2013
9. M. A. Pleitez Rafael, O. Hertzberg, A. Bauer, M. Seeger, T. Lieblein, H. von Lilienfeld-Toal, and W. Mäntele. Photothermal deflectometry enhanced by total internal reflection enables non- invasive glucose monitoring in human epidermis. The Analyst, November 2014.

Es besteht die Aufgabe, eine Vorrichtung und ein Verfahren anzugeben, mit der sich ein Stoff, insbesondere ein tierisches oder menschliches Gewebe oder ein Bestandteil oder Inhaltsstoff des Gewebes oder eine Flüssigkeit besonders einfach, präzise und kostengünstig analysieren lässt. Ein Aspekt der Erfindung liegt dabei auch bei der Erreichung einer geringen Baugröße der Vorrichtung.

Zudem wird auf die deutsche Patentschrift DE 10 2014 108 424 B3, sowie auch US2014/0114187A1 und WO2017/097824A1, verwiesen.

Diese Aufgabe wird unter anderem durch eine Vorrichtung mit den Merkmalen gemäß Anspruch 1 gelöst. Ausgestaltungen der Vorrichtung sind in Unteransprüchen angegeben. Zudem bezieht sich die Erfindung auf ein Verfahren gemäß dem unabhängigen Verfahrens-Patentanspruch 14 mit entsprechenden Ausgestaltungen gemäß dem/den von diesem abhängigen Unteranspruch/-ansprüchen.

Unter dem Begriff "Licht" oder "Laserlicht" werden im Zusammenhang der nachfolgenden Ausführungen elektromagnetische Wellen bzw. elektromagnetische Strahlung im sichtbaren Bereich, im nahen, mittleren und fernen Infrarotbereich sowie im UV-Bereich verstanden. Im Folgenden wird zunächst auf die bei Einreichung aufgeführten Anspruchsgegenstände eingegangen.

Die Aufgabe wird mit den Merkmalen der Erfindung gemäß Patentanspruch 1 gelöst.

Unter einer Phasenverschiebung des Detektionslichts ist in diesem Zusammenhang eine Phasenverschiebung gegenüber der Phasenlage des Detektionslichts vor der oder ohne die Temperatur- oder Druckänderung zu verstehen. Aus der Veränderung der Lichtintensität kann somit eine Phasenverschiebung des Detektionslichtes und aus dieser eine Änderung des Brechungsindex ermittelt werden. Aus der Änderung des Brechungsindex kann beispielsweise die Intensität einer Wärme- und/oder Druckwelle ermittelt werden, aus der ihrerseits in bevorzugten Ausführungsformen eine Absorptionsstärke und aus dieser die Konzentration einer nachzuweisenden Substanz ermittelt werden kann. Unter dem Begriff Detektionslicht kann außer sichtbarem Licht auch Infrarot- oder UV- Licht oder eine andere durch die Lichtwellenleiterstruktur führbare Art von elektromagnetischen Wellen verstanden werden.

Mittels des oder der Anregungsstrahlen wird Energie in den Stoff eingestrahlt und in Abhängigkeit von der eingestrahlten Licht-Wellenlänge und den in dem zu analysierenden Stoff vorhandenen Substanzen sowie deren Resonanzschwingungen bzw Absorptionsfrequenzen werden die Anregungsstrahlen in dem Stoff in höherem oder geringerem Maß absorbiert, wobei Wärmeenergie in Form von Molekülschwingungen freigesetzt wird. Die bezüglich der Wellenlänge durchstimmbaren Lichtquelle kann außer durch einen durchstimmbaren Laser oder ein Laserarray auch durch eine andersartige Strahlungsquelle gebildet sein, beispielsweise eine breitbandige Lichtquelle, aus der einzelne Wellenlängen wahlweise durch Filter ausselektiert werden können. Es können auch beispielsweise eine oder mehrere im Infrarotbereich strahlende Leuchtdioden verwendet werden, deren Strahlung schmalbandig auf gewünschte Wellenlängenbereiche selektierbar ist. Auch hier kann eine Modulation in der Lichtquelle oder im Lichtweg stattfinden.

Der Erwärmungsvorgang folgt bezüglich seiner Intensität der Modulation des Anregungsstrahls und erzeugt eine Wärme- und/oder Druckwelle, die sich in dem zu analysierenden Stoff unter anderem zur Messfläche hin und weiter in dem Messkörper ausbreitet und in der Detektionsvorrichtung die erste Lichtwellenleiterstruktur beeinflusst. Der Messkörper ist im Bereich der Messfläche mit dem Stoff gekoppelt, so dass eine Wärme- und/oder Druckwelle von dem Stoff auf den Messkörper übergehen kann. Die Kopplung kann unmittelbar durch physischen Kontakt zwischen dem Stoff und dem Messkörper, jedoch beispielsweise auch unter Zwischenfügung eines geeigneten festen oder fluiden, gasförmigen oder flüssigen Mediums erfolgen. Auf diese Weise kann die Kopplung beispielsweise auch in der Abstrahlung einer akustischen Druckwelle von dem Stoff zum Messkörper, gegebenenfalls auch eine Strecke weit durch ein gasförmiges Medium erfolgen. Durch geeignete Wahl von Medien zwischen dem Stoff und dem Messkörper kann eine Impedanzanpassung zur möglichst guten Einkopplung in den Messkörper vorgesehen sein.

Der Anregungsstrahl wird vorteilhaft in einem Bereich in den Stoff eingestrahlt, der entweder unmittelbar an der Messfläche anliegt oder mit dieser auf andere Weise gekoppelt ist. Der Anregungsstrahl kann auch unmittelbar neben einem Bereich der Messfläche, die mit dem zu analysierenden Stoff gekoppelt ist oder in Kontakt steht, in den Stoff eingestrahlt werden. Der Anregungsstrahl kann dabei durch das Volumen, durch eine Ausnehmung oder Bohrung im Messkörper oder insbesondere auch wenigstens abschnittsweise durch das Material des Messkörpers eingestrahlt werden oder auch an einer äußeren Begrenzung des Messkörpers vorbei in unmittelbarer Nähe des Messkörpers. Wird eine Ausnehmung/Bohrung im Messkörper für den Anregungsstrahl vorgesehen, so kann diese ganz durch den Messkörper hindurchgehen oder als Sackbohrung ausgebildet sein und in diesem Fall kann im Bereich der Messfläche das Material des Messkörpers oder auch eine Beschichtung aus einem anderen Material beispielsweise in einer Dicke von 0,05 mm bis 0,5 mm, insbesondere einer Dicke von 0,1 mm bis 0,3 mm stehen bleiben .

Durch die Beeinflussung der ersten Lichtleiterstruktur durch die Wärme- und/oder Druckwelle wird zumindest abschnittsweise in der ersten Lichtwellenleiterstruktur der Brechungsindex geändert und eine Phasenverschiebung des Detektionslichtes verursacht, die zu einer messbaren Änderung der Lichtintensität zumindest in einem Abschnitt der ersten Lichtwellenleiterstruktur führt.

Zum Nachweis solcher Phasenverschiebungen stehen beispielsweise interferometrische Verfahren und Vorrichtungen zur Verfügung.

Die Beschreibung bezieht sich demnach auch auf die Verwendung eines interferometrischen Messverfahrens oder einer interferometrischen Messvorrichtung zur quantitativen Messung der Temperaturerhöhung in einem Material beim Durchlauf einer Wärme- und/oder Druckwelle.

Der Messkörper kann durch einen Trägerkörper gebildet sein, an dem die Detektionslichtquelle sowie die erste Lichtwellenleiterstruktur befestigt oder angeordnet sein kann. Die Detektionslichtquelle kann entweder unmittelbar vor einer Einkoppelstelle der ersten Lichtwellenleiterstruktur angeordnet oder mit dieser mittels eines Lichtwellenleiters verbunden sein. Die Detektionslichtquelle kann auch unmittelbar in die Lichtwellenleiterstruktur als integriertes Halbleiterelement mit integriert, beispielsweise auf demselben Substrat wie die Lichtwellenleiterstruktur angeordnet sein. Der Lichtwellenleiter kann als faseroptischer Lichtwellenleiter oder auch als integrierter Lichtwellenleiter ausgebildet sein. Der Messkörper kann beispielsweise auch selbst ein Substrat darstellen oder enthalten, auf dem integrierte Lichtwellenleiter angeordnet werden können. Das Material des Messkörpers kann für das Anregungslicht transparent oder nicht transparent gestaltet sein. Die Messfläche kann als die äußere Begrenzungsfläche des Messkörpers definiert sein, die mit dem zu analysierenden Stoff gekoppelt oder in Kontakt gebracht werden kann, wobei eine Wärme- und/oder Druckwelle von dem Stoff durch die Messfläche hindurch zum Messkörper transportiert werden kann.

Bei der Gestaltung des Messkörpers kann vorgesehen sein, dass die erste Lichtwellenleiterstruktur so in Bezug auf die Messfläche angeordnet ist, dass sie von Druck- oder Wärmewellen beeinflusst wird, die auf Absorption des Anregungslichts zurückzuführen sind, wenn der Messkörper im Bereich der Messfläche mit dem Stoff gekoppelt/in Kontakt ist.

Beispielsweise kann vorgesehen sein, dass zumindest ein Abschnitt einer Projektion der ersten Lichtwellenleiterstruktur in Richtung der Flächennormalen der Messfläche mit dieser Messfläche überlagert ist.

Es kann auch allgemeiner vorgesehen sein, dass zumindest ein Abschnitt der ersten Lichtwellenleiterstruktur von der Messfläche aus, insbesondere von dem Bereich der Messfläche aus, in dem diese von dem Anregungsstrahl durchsetzt wird, in gerader Richtung durch eine Welle erreichbar ist.

Dabei ist es vorteilhaft, wenn zumindest ein Abschnitt der Lichtwellenleiterstruktur, insbesondere ein interferometrisches Element, weiter insbesondere wenigstens ein Arm eines Interferometers der Lichtwellenleiterstruktur innerhalb eines gedachten Kegels liegt, dessen Achse senkrecht zur Messfläche steht, dessen Spitze an der Stelle liegt, an der der Anregungsstrahl die Messfläche durchsetzt und der einen Öffnungswinkel von höchstens 90°, vorzugsweise höchstens 60° und besonders vorzugsweise höchstens 20° hat. Dabei bezeichnet der Öffnungswinkel das Zweifache des Winkels zwischen der Kugelachse und einer Mantellinie des gedachten Kegels.

Zudem kann vorgesehen sein, dass zumindest ein Abschnitt der ersten Lichtwellenleiterstruktur weniger als 2 mm, vorzugsweise weniger als 1 Millimeter, weiter vorzugsweise weniger als 0,5 mm von der Messfläche entfernt ist.

Es soll erreicht werden, dass die erste Lichtwellenleiterstruktur so in Bezug auf die Messfläche angeordnet ist, dass Wärme- und/oder Temperaturwellen, die durch Absorption des Anregungslichts in dem Stoff hervorgerufen werden, wenn der Messkörper im Bereich der Messfläche mit dem Stoff in Kontakt gekoppelt ist, zu einer messbaren Phasenverschiebung von Detektionslicht in wenigstens einem Teil der ersten Lichtwellenleiterstruktur führen.

Die Messfläche kann als ebene Fläche gestaltet sein, jedoch auch eine konkave Fläche oder Teilfläche aufweisen, auf der ein aufgelegter Körper oder Gegenstand gut zentriert oder positioniert werden kann. Die Messfläche kann dann beispielsweise die Form einer teilzylindrischen Rinne oder eine Kalottenform, insbesondere eine Kugelkalottenform aufweisen, wobei der Krümmungsradius beispielsweise zwischen 0,5 cm und 3 cm liegen kann, insbesondere zwischen 0,5 cm und 1,5 cm. Ist die Messfläche nicht vollständig eben, so soll als Flächennormale der Messfläche entweder die Flächennormale in der Mitte einer konkaven Ausnehmung der Messfläche oder die Flächennormale einer ebenen Oberfläche eines Körpers verstanden werden, die an die Messfläche angelegt ist. Als Flächennormale kann auch die Flächennormale einer ebenen Fläche verstanden werden, die unter Überbrückung der konkaven Ausnehmung der Messfläche deren Fortsetzung bildet.

Der Messkörper kann im Bereich der Messfläche auch eine Beschichtung mit einem Material aufweisen, das eine Wärme- und/oder Druckwelle möglichst verlustfrei leitet. Dieses Material kann beispielsweise gelartig oder fest sein und es kann auch für den Anregungsstrahl transparent sein oder in einem Bereich, in dem der Anregungsstrahl die Messfläche durchsetzt, eine Ausnehmung aufweisen. Die Beschichtung kann beispielsweise eher dünn sein mit einer Dicke, die geringer als 1 mm oder geringer als 0,5 mm ist, oder sie kann eher dick sein mit einer Dicke, die größer als 0,5 mm, insbesondere größer als 1 mm, weiter insbesondere größer als 2 mm ist.

Die oben genannten gekrümmten Oberflächenformen sind durch ein Substrat des Messkörpers gebildet, wobei eine gleichmäßig dicke Beschichtung vorgesehen sein kann, oder das Substrat kann eine ebene Oberfläche aufweisen, wobei eine gekrümmte Oberfläche durch das Dickenprofil der Beschichtung realisiert sein kann.

Die erste Lichtwellenleiterstruktur kann auf der der Messfläche gegenüberliegenden Seite des Messkörpers oder auf der der Messfläche zugewandten Seite des Messkörpers an dessen Oberfläche angeordnet sein. In diesem Fall kann der Messkörper ein Substrat bilden, auf dessen der Messfläche gegenüberliegenden Seite oder direkt unter der Messfläche Lichtwellenleiter aufgebracht sind, beispielsweise mittel Epitaxial-Aufdampftechnologie.

Die erste Lichtwellenleiterstruktur kann auch im Inneren des Messkörpers oder Substrats angeordnet und auf allen Seiten vom Material des Messkörpers/Substrats umgeben sein, um beispielsweise eine gute Zuleitung sowie guten Abtransport von Wärme oder auch Druckwellen zu gewährleisten. In diesem Fall kann die erste Lichtwellenleiterstruktur durch ein an sich bekanntes Herstellungsverfahren im Inneren eines Substrats "vergraben" sein, das heißt, sie ist allseitig von andersartigem Material bedeckt, das insbesondere einen anderen Brechungsindex aufweist als ein Lichtwellenleiter der ersten Lichtleiterstruktur selbst. Ist der Lichtwellenleiter selbst durch Silizium gebildet, so kann er beispielsweise durch Siliziumoxid bedeckt sein. Das Substrat /der Messkörper kann auch ganz oder teilweise aus Silizium bestehen. Es ist auch ein Aufbau der integrierten Lichtwellenleiter aus Kunststoff, beispielsweise aus Polyethylen oder aus einem optisch transparenten kristallinen Werkstoff denkbar. Die erste Lichtwellenleiterstruktur kann beispielsweise parallel zur Messfläche angeordnet und/oder in einer Ebene angeordnet sein, die parallel zur Messfläche ist. Grundsätzlich können die optisch integrierten Lichtwellenleiter beispielsweise als sogenannte strip- oder slot-Wellenleiter ausgeführt sein, das heisst, als Materialstreifen (strips), in denen Lichtwellen geführt werden, oder als passend geformte Zwischenräume (slots) zwischen totalreflektierenden Begrenzungen, die aus einem definierten Begrenzungsmaterial bestehen.

Bei einer Einrichtung der erläuterten Art ist vorgesehen, dass eine Modulationseinrichtung zur Intensitätsmodulierung des Anregungsstrahls vorgesehen ist.

Die Modulationseinrichtung kann die Intensität des Anregungsstrahls durch mechanisches Blockieren (mechanischer Chopper) steuern oder durch eine steuerbare Blenden- oder Ablenkspiegelvorrichtung oder einen Körper/eine Schicht, deren Transmission steuerbar ist. Zudem kann eine Modulation auch unmittelbar durch die Ansteuerung der Anregungslichtquelle/Laserlichtquelle erreicht werden oder durch eine Blende oder eine elektronische Intensitätssteuerung, die den Anregungsstrahl auf seinem Weg von der Anregungslichtquelle/der Lasereinrichtung zu dem zu analysierenden Stoff ganz oder teilweise blockiert oder ablenkt. Dies kann auch durch eine interferometrische Einrichtung oder einen elektronisch steuerbaren Piezokristall oder einen Flüssigkristall geschehen oder eine andere, elektronisch ansteuerbare Einrichtung, die die Transparenz oder Reflektivität für den Anregungslichtstrahl verändert. Eine solche Einrichtung kann als integraler Bestandteil der Lasereinrichtung vorgesehen sein oder auch als ein Funktionselement, das in den Messkörper/das Substrat funktional mit integriert ist. Dies ist dadurch möglich, dass durch Gestaltung des Substrats dreidimensionale Funktionsstrukturen der integrierten Optik und der Elektronik durch ein- oder mehrschichtige Gestaltung gebildet werden können. Auch MEMS-Strukturen (Mikro-elektromechanische Strukturen) können auf diese Weise in das Substrat mit integriert werden, um beispielsweise einen steuerbaren Ablenkspiegel zur Lichtmodulation zu schaffen. Ein möglicher Aspekt des hier vorgestellten Verfahrens ist die Fokussierung der Messung des Reaktionssignals auf selektierte Tiefenbereiche unterhalb der (Abstandsintervalle von der) Stoffoberfläche. Die Größe d hat dabei den größten Einfluss auf den mit dem Verfahren gemessenen Tiefenbereich. Sie ist definiert als d = √(D/(π*f)), wobei D die thermische Diffusivität der Probe (hier bspw. Haut) ist und f die Modulationsfrequenz des Anregungstrahls. Bezüglich weiterer Einzelheiten zur thermischen Diffusivität von Haut wird auf die folgenden Schriften verwiesen:
- U. Werner, K. Giese, B. Sennhenn, K. Plamann, and K. Kölmel, "Measurement of the thermal diffusivity of human epidermis by studying thermal wave propagation," Phys. Med. Biol. 37(1), 21-35 (1992).
- A. M. Stoll, Heat Transfer in Biotechnology, Vol 4 of Advances in Heat Transfer, J. P. Hartnett and T. Irvin, eds. (New York, Academic, 1967), p 117.

Man beachte, dass in der vorliegenden Offenbarung derselbe Begriff "Reaktionssignal" in mehrfacher Hinsicht verwendet wird. Zum einen kann er die physikalische Antwort auf die Anregung durch den Anregungsstrahl bezeichnen, also beispielsweise eine Schallwelle, eine Erwärmung oder dergleichen. Andererseits kann er aber auch ein optisches oder elektrisches Signal bezeichnen, welches diese physikalische Antwort repräsentiert, also beispielsweise die Intensität des Detektionslichts (als Beispiel eines optischen Signals), bzw. einen Messwert der Intensität, bei dem es sich um ein elektrisches Signal handelt. Zur Einfachheit und Kohärenz der Darstellung wird stets derselbe Begriff "Reaktionssignal" verwendet, wobei aus dem Zusammenhang ohne weitere Erläuterung ersichtlich ist, ob es sich dabei um die physikalische Antwort (beispielsweise eine Druckwelle oder Temperaturwelle), eine physikalische Folge dieser physikalischen Antwort (beispielsweise eine Phasenverschiebung des Detektionslichts) oder das zugehörige Messsignal (beispielsweise die mit einem Photosensor gemessene Intensität des Detektionslichts) handelt.

Zur Eliminierung von Reaktionssignalen aus den obersten Schichten des Stoffes zum Zweck der Qualitätsverbesserung der Messung können in einer Ausführungsform Änderungen der Messwerte im Vergleich zu vorangegangenen Messungen herangezogen werden, falls die Messwerte in den obersten Schichten sich im Vergleich zu anderen, tieferliegenden Schichten weniger oder langsamer verändern. Dies kann in einer Ausführungsform bei Messungen an der menschlichen Haut der Fall sein, wo die obersten Hautschichten praktisch keinem Austausch mit den unteren Schichten unterliegen und deshalb physiologische Parameter wenig veränderlich sind. Es kann auch die zeitliche Ableitung von Messwerten zu Reaktionssignalen zum Ausschluss der Signale aus den obersten Hautschichten herangezogen werden. So kann die Messung oder zumindest die Auswertung auf die interstitielle Flüssigkeit in der Haut begrenzt oder fokussiert werden.

Zu diesem Zweck kann eine Messung die Aufnahme von Reaktionssignalen für Spektren umfassen, die mehrfach mit verschiedenen Modulationsfrequenzen der Anregungslichtquelle erfasst werden, wobei die Ergebnisse für verschiedene Modulationsfrequenzen miteinander verknüpft werden, beispielsweise durch Differenzbildung oder Quotientenbildung der Messwerte von Reaktionssignalen für gleiche Wellenlängen und verschiedene Modulationsfrequenzen. Es sollte zur Durchführung einer solchen Messung auch eine Vorrichtung mit einer entsprechenden Steuereinrichtung für den Anregungsstrahl und einer Auswerteeinrichtung für die Spektren von Reaktionssignalen vorgesehen sein.

Es ist weiter eine Messeinrichtung vorgesehen zur direkten oder indirekten Erfassung der Lichtintensität in der ersten Lichtwellenleiterstruktur, in einem Abschnitt, in dem die Lichtintensität von einer Phasenverschiebung von Detektionslicht in wenigstens einem Teil der ersten Lichtwellenleiterstruktur durch eine Temperatur- oder Druckänderung abhängt. Die Messeinrichtung kann eine Lichtintensität in der ersten Lichtwellenleiterstruktur selbst messen oder die Intensität eines an einer Koppelstelle ausgekoppelten Detektionslichtanteils. Die Messeinrichtung kann ein in das Substrat integriertes lichtempfindliches Halbleiterelement wie beispielsweise eine Photodiode umfassen. Damit kann direkt eine Lichtintensität gemessen werden. Indirekte Messmethoden können beispielsweise durch Messung anderer Parameter wie der Temperatur oder einer Feldstärke an der ersten Lichtwellenleiterstruktur gegeben sein.

Weiter kann vorgesehen sein, dass die Detektionsvorrichtung eine interferometrische Einrichtung, insbesondere ein Interferometer und/oder einen Lichtwellenleiter-Resonanzelement, insbesondere einen Resonanzring oder eine Resonanzplatte aufweist. Als interferometrische Einrichtung kann dabei ein Interferometer, insbesondere ein Mach-Zehnder- Interferometer vorgesehen sein, bei dem das Detektionslicht durch einen Strahlteiler in zwei Teilstrahlen aufgespalten wird, die über zwei separate Arme des Interferometers geleitet werden. Die beiden Arme des Interferometers sind der Wirkung der Temperatur- und/oder Druckwelle unterschiedlich stark ausgesetzt, wobei der Messarm der Wirkung der Temperatur- und/oder Druckwelle stärker ausgesetzt ist als der Referenzarm oder der Einfluss der Temperatur-und/oder Druckwelle bezüglich einer Änderung des Brechungsindex beim Messarm stärker ist als beim Referenzarm. Im optimalen Fall ist der Referenzarm von der Wirkung der Temperatur- und/oder Druckwelle völlig unbeeinflusst, während der Messarm der Wirkung voll ausgesetzt ist.

Außer einer direkten Messung einer Lichtintensität in den oben beschriebenen Varianten kann auch indirekt mittels eines anderen Parameters wie beispielsweise einer Temperatur oder Feldstärke die Intensität des Detektionslichts gemessen werden, sofern der zu messenden Parameter von der Lichtintensität abhängt.

Um sicher zu stellen, dass der Messarm der Wirkung der Temperatur und/oder Druckwelle stärker angesetzt ist, kann beispielsweise vorgesehen sein, dass zumindest ein Abschnitt einer Projektion des Messarms der ersten Lichtwellenleiterstruktur in Richtung der Flächennormalen der Messfläche mit dieser Messfläche überlagert ist.

Zudem kann für eine hohe Effizienz der Messung vorgesehen sein, dass zumindest ein Abschnitt des Messarms der ersten Lichtwellenleiterstruktur weniger als 2 mm, vorzugsweise weniger als 1 Millimeter, weiter vorzugsweise weniger als 0,5 mm von der Messfläche entfernt ist. Der Referenzarm kann von der Messfläche weiter entfernt sein als der Messarm, wie an anderer Stelle in dieser Anmeldung genauer beschrieben ist.

Es soll erreicht werden, dass der Messarm der ersten Lichtwellenleiterstruktur so in Bezug auf die Messfläche angeordnet ist, dass Wärme- und/oder Temperaturwellen, die durch Absorption des Anregungslichts in dem Stoff hervorgerufen werden, wenn der Messkörper im Bereich der Messfläche mit dem Stoff in Kontakt ist, zu einer messbaren Phasenverschiebung von Detektionslicht in wenigstens einem Teil des Messarms der ersten Lichtwellenleiterstruktur führen. Der Messarm und/oder der Referenzarm eines Interferometers kann vorteilhaft parallel zur Messfläche ausgerichtet sein und/oder in einer Ebene verlaufen, die parallel zur Messfläche liegt.

Das Licht aus beiden Armen wird nach Durchlaufen der Arme wieder zusammengeführt und in Abhängigkeit von der Phasenverschiebung des Detektionslichtes in dem Arm, der der Wirkung stärker ausgesetzt ist, löschen sich die beiden gegeneinander phasenverschobenen Teilstrahlen des Detektionslichtes wenigstens teilweise aus. Die gemessene Lichtintensität ist dann minimiert, es sei denn, die Phasenverschiebung überschreitet 180 Grad und durchläuft im Extremfall mehrere volle Zyklen von je 360 Grad (2 Pi). In diesem Fall können im Zuge der Entfaltung der Temperatur- und/oder Druckerhöhung auch die Nulldurchgänge bei der Phasenauslöschung der beiden Teilstrahlen gezählt werden, um eine absolute Phasenverschiebung zu bestimmen. In vielen Fällen wird die Phasenverschiebung jedoch aufgrund der geringen nachzuweisenden Temperatur- und/oder Druckeffekte 180 Grad nicht überschreiten. Der Arbeitspunkt des Interferometers kann dann so eingestellt werden, dass die auftretenden Lichtintensitätsänderungen auf die Druck/Temperaturänderungen monoton abgebildet werden.

Durch folgende Maßnahmen kann bewirkt werden, dass der Messarm der Wirkung der Temperatur-und/oder Druckwelle stärker ausgesetzt ist als der Referenzarm, oder dass der Einfluss der Temperatur-und/oder Druckwelle bezüglich einer Änderung des Brechungsindex beim Messarm stärker ist als beim Referenzarm:
Der Messarm, mit oder ohne einen in diesen integrierten oder mit diesem verbundenen Ringresonator steht in mechanischem Kontakt mit dem Substrat. Der Lichtwellenleiter des Messarms kann formschlüssig und/ oder stoffschlüssig und/oder kraftschlüssig mit dem Substrat verbunden sein. Er kann auch gegen das Substrat gedrückt oder an diesem eingeklemmt werden.

Enthält die interferometrische Einrichtung nur einen oder mehrere Ringresonatoren oder andere Lichtwellenleiter-Resonanzelemente, so können diese ebenso mit dem Substrat in mechanischem Kontakt stehen. Der/die Lichtwellenleiter des/der Ringresonator(en) oder Resonanzelemente können ebenso formschlüssig und/oder stoffschlüssig und/oder kraftschlüssig mit dem Substrat verbunden sein. Sie können auch gegen das Substrat gedrückt oder an diesem eingeklemmt werden.

Ein Interferometer oder einer oder beide seiner Messarme kann bzw. können ebenso wie ein oder mehrere Ringresonatoren oder andere Resonanzelemente fertigungstechnisch in das Substrat integriert sein und beispielsweise gemeinsam mit diesem in einem einheitlichen Fertigungsprozess hergestellt sein.

Eine reduzierte Wirkung der Temperatur- und/oder Druckwelle auf den Referenzarm oder eine reduzierter Einfluss auf den Brechungsindex des/der Lichtwellenleiter oder des optischen Lichtweges im Referenzarm kann dabei unter anderem dadurch realisiert sein, dass wenigstens ein Teil des Lichtwellenleiters oder auch der gesamte Lichtwellenleiter des Referenzarms aus einem faseroptischen Lichtwellenleiter besteht und in diesem Fall der faseroptische Lichtwellenleiter abschnittsweise oder auf dem überwiegenden Teil seiner Länge oder vollständig außerhalb des Substrats, insbesondere von diesem beabstandet angeordnet ist. Der faseroptische Lichtwellenleiter kann auch außerhalb des Materials des Substrats, beispielsweise in einer Ausnehmung des Substrats verlaufen, ohne mit dem Material des Substrats verbunden zu sein.

Es kann auch wenigstens ein Teil des Referenzarms separat von dem Messarm durch ein zweites Substrat, an einem zweiten Substrat, oder in oder an einem von dem Substrat zumindest abschnittsweise getrennten oder abgeschirmten oder beabstandeten Substratteil verlaufen.

In diesem Fall kann der Referenzarm oder das zweite Substrat oder das Teilsubstrat von dem Substrat wenigstens abschnittsweise durch einen Luftspalt oder eine Barriere getrennt sein. Als Barriere kommen Stoffe in Frage, die weicher oder weniger steif sind als das Substratmaterial und beispielsweise aus einem Kunststoff, einem Elastomer, einem organischen Material, einem Textil, Papier oder einem Schaumstoff bestehen.

In jedem Fall kann beispielsweise wenigstens 10%, insbesondere wenigstens 20%, weiter insbesondere wenigstens 30% der optischen Länge des Referenzarms in einem Bereich desselben Substrats wie der Messarm oder eines weiteren Substats angeordnet sein, der von dem Messarm einen Abstand von wenigstens 2 mm, insbesondere wenigstens 5 mm, weiter insbesondere wenigstens 8mm aufweist. Dabei kann dieser Bereich des Referenzarms vorzugsweise von der Messfläche weiter entfernt sein als der Messarm. Der genannte Anteil des Referenzarms kann vorteilhaft in einem Bereich liegen, der von der Wärme- und/oder Druckwelle nicht erreicht wird oder zumindest weniger beeinflusst wird als der Bereich in dem der Messarm liegt.

Wenn der Messarm und der Referenzarm wenigstens teilweise aus unterschiedlichen Materialien bestehen, kann ein Strahlteiler zur Aufteilung des Detektionslichts auf den Messarm und den Referenzarm vorgesehen sein. Dieser Strahlteiler kann in das Substrat integriert sein oder separat zu diesem vorgesehen sein. Der Strahlteiler kann derart gestaltet sein, dass er das Detektionslicht auf einen integrierten Lichtwellenleiter und einen faseroptischen Lichtwellenleiter, oder auf zwei integrierte Lichtwellenleiter oder auf zwei faseroptische Lichtwellenleiter verteilt.

Unabhängig von der Anordnung und dem Abstand des Messarms und des Referenzarms zueinander können der Messarm und der Referenzarm wenigstens teilweise oder vollständig aus unterschiedlichen Materialien bestehen, wobei das Material des Referenzarms so gewählt sein kann, dass sein Brechungsindex durch den Einfluss der Wärme- und/oder Druckwelle weniger beeinflusst wird als der Brechungsindex des Materials des Messarms. Dies kann beispielsweise durch die Wahl unterschiedlicher Grundmaterialien für Messarm und Referenzarm verwirklicht sein, oder auch durch unterschiedliche Dotierung desselben Grundmaterials im Mess- und Referenzarm. Es kann auch vorgesehen sein, dass das Detektionslicht wenigstens auf einem Teil der Strecke des Referenzarms durch ein Fluid, insbesondere ein Gas, beispielsweise Luft oder Stickstoff oder eine transparente Flüssigkeit geleitet wird.

Wird als Detektionsvorrichtung ein Ringresonator oder ein anderes Lichtwellenleiter- Resonanzelement verwendet, so kann dieser oder dieses in einer Ebene angeordnet sein, die parallel zur Messfläche liegt. Damit werden alle Abschnitte des Ringresonators möglichst gleichmäßig der Wirkung einer von der Messfläche auf den Ringresonator fallenden Temperatur- und/oder Druckwelle ausgesetzt. Wird als Detektionsvorrichtung ein Ringresonator oder ein anderes Resonanzelement verwendet, entweder ausschließlich oder verknüpft mit einem Interferometer, so kann statt eines einzelnen Ringresonators oder Resonanzelementes auch eine Mehrzahl optisch hintereinandergeschalteter oder parallelgeschalteter Ringresonatoren oder Resonanzelemente verwendet werden, um den Frequenzgang wunschgemäß zu gestalten. Der oder die Arbeitspunkte können durch eine Temperaturregelung oder durch Einstellung eines mechanischen Drucks auf die Ringresonatoren/Resonanzelemente eingestellt werden. Die Arbeitspunkteinstellung kann derart erfolgen, dass eine maximale Temperatur- oder Druckempfindlichkeit oder ein maximaler Messbereich mit einer monotonen Abhängigkeit zwischen Temperatur oder Druck und der Lichtintensität im Resonanzring/Resonanzelementesgegeben ist.

Die Einrichtung zur Analyse eines Stoffes kann eine Auswerteeinrichtung umfassen, die die durch die Detektionseinrichtung erfasste Intensitätsänderung des Detektionslichtes auswertet und aus dieser eine Absorptionsstärke in Abhängigkeit von den Wellenlängen des Anregungsstrahls ermittelt. Dabei kann aufgrund der Modulation des Anregungsstrahls die Detektionslichtintensität mit und ohne den Einfluß der zu messenden Wärme- und/oder Druckwelle erfasst und deren Differenz oder Verhältnis oder eine andere Beziehungsgröße zwischen diesen Werten ausgewertet werden.

Die Auswerteeinrichtung kann, insbesondere wenn als interferometrisches Element ein Lichtwellenleiter-Resonanzelement vorgesehen ist oder ein Interferometer mit zwei Messarmen mit Messabschnitten, die so relativ zur Messfläche angeordnet und orientiert sind, dass sie von der Wärme- und/oder Druckwelle nacheinander erreicht werden, auch derart eingerichtet sein, dass der Verlauf der Intensität des Detektionslichtes, also der Verlauf der Verstimmung des Resonanzelementes durch Änderung des Brechungsindex oder der Verlauf der Phasenverschiebung in den beiden Messabschnitten/Messarmen des Interferometers während des Durchlaufens einer Wärme- und/oder Druckwelle oder einer oder mehrerer Wellenfronten erfasst wird.

Insbesondere bei Verwendung eines Interferometers mit mehreren Messabschnitten können sich, wenn diese beide der Wärme- und/oder Druckwelle ausgesetzt sind, die Phasenverschiebungen ausgleichen, so dass keine Änderung der Detektionslichtintensität zu beobachten ist. Sind die Messarme/Messabschnitte jedoch so positioniert /orientiert, dass sie nacheinander von der Welle erreicht werden, so wird sich ein Intensitätsverlauf des Detektionslichtes einstellen, der die unterschiedliche und zeitlich versetzte Wirkung der Welle auf die verschiedenen Messabschnitte wiederspiegelt und damit eine Auswertung erlaubt, da es Zeitabschnitte gibt, in denen die Welle auf die verschiedenen Messabschnitte unterschiedlich wirkt.

Bei den Lichtwelleneiter-Resonanzelementen ergibt sich ein zeitlicher Verlauf der Intensität des Detektionslichtes, der die Amplitude der durchlaufenden Welle wiederspiegelt.

Es kann dann bei einem modulierten Anregungsstrahl und den entsprechend durchlaufenden Wärme- und/oder Druckwellen ein passender Parameter, beispielsweise die Amplitude, der periodischen Änderung der Intensität des Detektionslichtes zur Auswertung herangezogen werden.

Es kann weiter vorgesehen sein, dass die Lichtwellenleiterstruktur, insbesondere die interferometrische Einrichtung der ersten Lichtwellenleiterstruktur, wenigstens einen Glasfaser-Lichtwellenleiter aufweist, der wenigstens abschnittsweise mit dem Messkörper fest verbunden ist.

Ein Glasfaser-Lichtwellenleiter ist kostengünstig verfügbar und durch seine Flexibilität gut an die bestehenden Bedürfnisse anpassbar. Er muss jedoch mit dem Messkörper in Kontakt gebracht werden, um durch die Druck- und/oder Wärmewelle beeinflusst zu werden. Dazu kann der Lichtwellenleiter mit dem Substrat/Messkörper verklebt oder mit diesem formschlüssig oder kraftschlüssig verbunden sein. Beispielsweise kann der Glasfaser- Lichtwellenleiter in einer Klemmeinrichtung des Substrats befestigt sein.

Es kann auch vorgesehen sein, dass ein Lichtwellenleiter der ersten Lichtwellenleiterstruktur, insbesondere einer interferometrischen Einrichtung der ersten Lichtwellenleiterstruktur, in ein Substrat des Messkörpers integriert oder mit einem Substrat verbunden ist, wobei die erste Lichtwellenleiterstruktur insbesondere wenigstens einen Silizium-Lichtwellenleiter aufweist, der mit einem isolierenden Substrat verbunden oder in ein isolierendes Substrat integriert ist, und wobei weiter insbesondere der Silizium- Lichtwellenleiter wenigstens teilweise durch einen Isolator, insbesondere ein Siliziumoxid, beispielsweise SiO2 abgedeckt ist.

In diesem Fall kann die erste Lichtwellenleiterstruktur auf dem Substrat mit den bekannten Mitteln der integrierten Optik aufgebaut werden, wobei Bereiche mit verschiedenem Brechungsindex beispielsweise durch selektive Dotierung des Substratmaterials oder durch die Ausbildung von Oxidschichten oder anderen Schichten aus Reaktionsprodukten geschaffen werden können. Solche integrierten Optischen Lichtwellenleiterstrukturen können in oder an einem Silizium-Wafer vorgesehen sein. Es kann auch eine Lichtwellenleiterstruktur in einem Polymerkörper gebildet werden. Außerdem können integrierte optische Lichtwellenleiter beispielsweise unter Verwendung der Materialkombinationen GeO2-SiO2/SiO2, GaAsInP/InP. Ti: LiNbO3 gebildet werden.

Zudem kann vorgesehen sein, dass der Anregungsstrahl das Material des Messkörpers, insbesondere im Bereich der Messfläche des Messkörpers oder einen der Messfläche benachbarten Bereich oder einen an die Messfläche unmittelbar angrenzenden Bereich durchsetzt, wobei der Messkörper oder der von dem Anregungsstrahl durchsetzte Bereich für den Anregungsstrahl transparent ist.

Dabei soll die Transparenz des Messkörpers und insbesondere auch einer Beschichtung des Messkörpers in dem Wellenlängenbereich des Anregungsstrahls oder Anregungslichtstrahls gegeben sein. Die Transparenz kann auch nicht vollständig gegeben sein, so dass eine gewisse Absorption des Anregungsstrahls in Kauf genommen wird. Die Schicht des Messkörpers, die von dem Anregungsstrahls durchsetzt wird, kann dann möglichst dünn, beispielsweise dünner als 1 mm, gestaltet werden, beispielsweise nur als dünne Schicht im Bereich der Messfläche.

Es kann auch vorgesehen sein, dass der Anregungsstrahl innerhalb des Messkörpers oder am Messkörper durch eine zweite Lichtwellenleiterstruktur geführt ist. Die zweite Lichtwellenleiterstruktur ist dann so gestaltet, dass sie Licht oder Strahlung im Wellenlängenbereich des Anregungsstrahls möglichst verlustfrei führen kann. Der Anregungsstrahl wird in einen Lichtwellenleiter der zweiten Lichtwellenleiterstruktur eingekoppelt und im Bereich der Messfläche aus diesem ausgekoppelt und auf den zu untersuchenden Stoff gerichtet. Dabei kann an der Einkoppelstelle und/oder an der Auskoppelstelle jeweils ein strahlformendes optisches Element, insbesondere ein fokussierendes oder kollimierendes Element vorgesehen sein, das separat von der Lichtwellenleiterstruktur vorgesehen oder in diese integriert sein kann. Die erste und die zweite Lichtwellenleiterstruktur können separat und voneinander getrennt und beabstandet vorgesehen sein. Sie können wegen der Linearität der Wellengleichung jedoch auch ohne eine Wechselwirkung einander überschneiden, so dass es in der/den Lichtwellenleiterstrukturen Bereiche gibt, die sowohl vom Anregungsstrahl als auch vom Detektionslicht durchlaufen werden. Im Extremfall können die erste und die zweite Lichtwellenleiterstruktur identisch sein und die notwendigen Ein- und Auskoppelstellen für den Anregungslichtstrahl sowie für das Detektionslicht aufweisen. Es ist auch denkbar, dass die Rückwirkung der Druck- und/oder Temperaturänderung in der Lichtwellenleiterstruktur auf das Anregungslicht detektiert und bei der Auswertung berücksichtigt wird. Die Lasereinrichtung zur Erzeugung des Anregungsstrahls kann in den Messkörper integriert sein und wenigstens ein oder mehrere oder alle elektronischen Elemente der Lasereinrichtung können auf einem Substrat des Messkörpers, insbesondere auf demselben Substrat vorgesehen sein, das auch die integrierten optischen Elemente trägt. Elektrische Elemente der Lasereinrichtung und integrierte optische Elemente können in einem gemeinsamen Herstellungsprozess und/oder in mehreren aufeinander folgenden Herstellungsschritten auf einem oder mehreren zusammenhängenden Substraten hergestellt oder angeordnet werden. Damit ergibt sich eine äußerst platzsparende Anordnung. Diese integrierte Anordnung kann sowohl dann vorgesehen sein, wenn für die Leitung des Anregungsstrahls eine zweite Lichtwellenleiterstruktur vorgesehen ist, als auch in dem Fall, dass der Anregungsstrahl durch eine Ausnehmung des Messkörpers auf den zu analysierenden Stoff gerichtet ist.

Es kann auch vorgesehen sein, dass der Anregungsstrahl zwischen der Lasereinrichtung und dem zu analysierenden Stoff eine durchgehende Ausnehmung des Messkörpers durchsetzt, wobei die Ausnehmung insbesondere in einem Abstand vor der Messfläche endet oder die Messfläche durchsetzt oder in einem Bereich angeordnet ist, der der Messfläche unmittelbar benachbart ist und/oder an diese angrenzt.

In diesem Fall breitet sich der Anregungsstrahl in der Ausnehmung aus und tritt in einigen Fällen auf der dem zu analysierenden Stoff zugewandten Seite des Messkörpers aus der Ausnehmung aus, ohne das Material des Messkörpers durchlaufen zu haben. Es kann auch im Bereich der Messfläche eine dünne Schicht des Messkörpers stehen bleiben, so dass die Ausnehmung nicht ganz durchgeht und in einem Abstand vor der Messfläche endet. Wichtig ist dabei, dass das Volumen des Stoffes, in das der Anregungsstrahl eingestrahlt wird, der Messfläche benachbart ist und mit dieser im Kontakt steht oder auf andere geeignete Weise gekoppelt ist, so dass die erzeugte Temperatur- und/oder Wärmewelle wenigstens teilweise die Messfläche trifft und durch diese hindurch in den Messkörper oder zur Detektionsvorrichtung geleitet wird.

Die durchgehende oder weitgehend durchgehende Ausnehmung in dem Messkörper kann einen geraden Kanal bilden, sie kann jedoch auch einen Kanal mit Krümmungen oder Knicken bilden, wobei der Anregungsstrahl dann mittels ablenkender oder reflektierender Elemente durch den Kanal geführt werden kann. Die Ausnehmung kann sich durch eine Beschichtung des Messkörpers hindurch fortsetzen, sie kann jedoch auch an der Beschichtung enden, so dass der Anregungsstrahl durch die Beschichtung geleitet wird.

Durchläuft der Anregungsstrahl zumindest eine bestimmte Schicht des Messkörpers, so kann, falls das Material des Messkörpers den Anregungsstrahl wenigstens teilweise absorbiert, durch den Anregungsstrahl bereits eine Temperaturerhöhung des Messkörpers erzeugt werden, die jedoch genau berechenbar ist. Durch den periodischen Betrieb der Anregungslichtquelle ergeben sich Wärmewellen in dem Messkörper, die in manchen Fällen die Detektionseinrichtung erreichen und durch diese nachgewiesen werden können. Dieser Einfluss kann berechnet und von dem Nutzsignal abgezogen werden.

Es kann auch vorgesehen sein, dass der Anregungsstrahl unmittelbar an einer äußeren Begrenzung des Messkörpers entlang auf den zu analysierenden Stoff gelenkt wird und in der Verlängerung der Messfläche neben dem Messkörper in den Stoff eindringt. Die erste Lichtwellenleiterstruktur der Detektionseinrichtung, beispielsweise ein Interferometer, kann dann unmittelbar neben dem Bereich, in dem der Anregungsstrahl die gedachte Fortsetzung der Messfläche durchsetzt, in dem Messkörper vorgesehen sein, so dass die dort aus dem Stoff austretende Wärme und/oder Druckwelle wenigstens teilweise in den Messkörper eintritt und die erste Lichtwellenleiterstruktur erreicht.

Eine weitere Ausführungsform kann vorsehen, dass der Messkörper als Flachkörper, insbesondere als planparalleler Körper in Form einer Platte ausgebildet ist, wobei insbesondere die Dicke des Messkörpers in Richtung senkrecht zur Messfläche weniger als 50% der geringsten Ausdehnung des Messkörpers in einer in der Messfläche verlaufenden Richtung beträgt, insbesondere weniger als 25%, weiter insbesondere weniger als 10%.

Eine solche Gestaltung kann sich aus der Verwendung eines flachen Substrats, beispielsweise eines Wafers, für die integrierte Optik ergeben. Die notwendige Dicke des Messkörpers ist dann durch den Raum begrenzt, der für die Detektionsvorrichtung benötigt wird.

Eine weitere Ausführungsform kann vorsehen, dass der Messkörper eine Spiegeleinrichtung zur Reflexion des von der Lasereinrichtung eingestrahlten Anregungsstrahls auf die Messfläche aufweist oder trägt.

Dies ist insbesondere dann wichtig, wenn die Lasereinrichtung so ausgerichtet ist, dass der Anregungsstrahl in der Lasereinrichtung nicht senkrecht zur Messfläche erzeugt wird, beispielsweise, wenn die Lasereinrichtung platzsparend neben dem Messkörper oder schräg zu diesem orientiert eingebaut werden soll.

Es kann auch vorgesehen sein, dass der Anregungsstrahl parallel zur Messfläche oder in einem Winkel von weniger als 30 Grad, insbesondere weniger als 20 Grad, weiter insbesondere weniger als 10 Grad oder weniger als 5 Grad zur Messfläche in den Messkörper ausgerichtet ist und dass der Anregungsstrahl in Richtung der Messfläche umgelenkt oder abgelenkt wird wobei der Anregungsstrahl insbesondere die Messfläche oder eine gedachte Fortsetzung der Messfläche im Bereich einer durchgehenden Ausnehmung in dem Messkörper durchsetzt.

In diesem Fall kann die Lasereinrichtung zur Erzeugung des Anregungsstrahls besonders platzsparend angeordnet und derart ausgerichtet sein, dass sie den Anregungsstrahl parallel zur Messfläche oder in einem der genannten flachen Winkel in Bezug auf die Messfläche erzeugt oder auskoppelt.

Außerdem kann vorgesehen sein, dass in dem Messkörper von der Messfläche aus gesehen hinter und/oder neben der Detektionsvorrichtung, insbesondere an diese angrenzend und mit dieser im thermischen Kontakt stehend wenigstens eine Wärmesenke in Form eines festen Körpers oder Materials angeordnet ist, wobei insbesondere die spezifische Wärmekapazität und/oder spezifische Wärmeleitfähigkeit des Körpers oder des Materials der Wärmesenke größer ist als die spezifische Wärmekapazität und/oder spezifische Wärmeleitfähigkeit des Materials der Detektionsvorrichtung und/oder der Lichtwellenleiterstruktur und/oder des Substrats der Lichtwellenleiterstruktur und/oder der übrigen Materialien, aus denen der Messkörper besteht.

Grundsätzlich kann es vorteilhaft sein, in oder an dem Messkörper eine Wärmesenke vorzusehen, um die Wärme, die durch eine Wärmewelle in die Detektionsvorrichtung eingebracht wird, möglichst schnell abzuführen, so dass auch bei hohen Modulationsfrequenzen des Anregungsstrahls ein Wärmeenergiegleichgewicht oder Temperaturgleichgewicht entsteht, das die Messung der intermittierend eingestrahlten Wärmemengen oder der hierdurch erzeugten Temperaturänderungen erlaubt, ohne dass die Messung durch Temperaturänderungen in der Vergangenheit verfälscht wird.

Bei einigen Anwendungen, insbesondere bei interferometrischen Anwendungen, weiter insbesondere bei einem Mach-Zehnder-Interferometer ist es zudem vorteilhaft, jeweils einen Messarm den Temperaturänderungen oder Druckwellen auszusetzen und den anderen Arm, den Referenzarm, von den Temperaturänderungen oder Druckwellen abzuschirmen. Zu diesem Zweck kann es auch sinnvoll sein, den Referenzarm von dem Messarm zu beabstanden und/oder eine Barriere vorzusehen, die den Einfluss der Wärme- und/oder Druckwelle von einem Teil der Detektionsvorrichtung, insbesondere dem Referenzarm eines Interferometers, wenigstens teilweise fernhält. Eine solche Barriere kann beispielsweise aus einem Material bestehen, das eine geringere Wärmeleitfähigkeit aufweist, als das Material des Messkörpers oder eines Substrats des Messkörpers. Das Material kann auch zur mechanischen Entkopplung von einer Druckwelle nachgiebiger oder elastischer oder leichter verformbar sein als das Material des Messkörpers oder eines Substrats des Messkörpers. Eine Barriere kann beispielsweise auch durch einen Gasspalt gebildet sein, der sich in ein Substrat beispielsweise durch Ätzen oder eine materialabtragende Bearbeitung oder auch durch ein additives Herstellungsverfahren einbringen lässt.

Zur Beabstandung des Referenzarms eines Mach- Zehnder- Interferometers von dem Messarm kann es auch vorgesehen sein, dass der Messarm und der Referenzarm in verschiedenen Ebenen des Substrats angeordnet sind, wobei die Ebene, in der sich der Referenzarm befindet, einen größeren Abstand von der Messfläche aufweist als die Ebene, in der der Messarm angeordnet ist.

Eine Temperatur- und/oder Druckänderung lässt sich auch durch einen LichtwellenleiterResonanzring erfassen, in dem sich das Detektionslicht bei geeigneten Verhältnissen in Resonanz ausbreitet. Bei einer Änderung von Temperatur- und/oder Druckverhältnissen wird durch Änderung des Brechungsindex die Resonanz verstimmt und es findet eine Teilauslöschung oder Auslöschung statt. Ein solcher Resonanzring weist eine Empfindlichkeit auf, die im Idealfall noch deutlich höher ist als die eines Mach- Zehnder-Interferometers. Ein solcher Resonanzring kann auch in einen Arm, vorzugsweise den Messarm eines Mach- Zehnder-Interferometers integriert werden.

Es kann in einer Ausführungsform der Erfindung auch vorgesehen sein, dass die Lichtwellenleiterstruktur der Detektionseinrichtung wenigstens zwei Mess-Abschnitte aufweist, die insbesondere an verschiedenen Armen eines Interferometers angeordnet sind und in denen der Brechungsindex sich in Abhängigkeit von Druck- und/oder Temperaturänderungen, insbesondere von einer Druck-und/oder Wärmewelle ändert, so dass eine Phasenverschiebung des die Mess-Abschnitte durchlaufenden Detektionslichts und in deren Folge eine Intensitätsänderung des Detektionslichts in einem weiteren Abschnitt in Abhängigkeit von Druck- und/oder Temperaturänderungen erfolgt, wobei die beiden Messabschnitte derart in dem Messkörper angeordnet sind, dass sie durch eine Druck-und/oder Wärmewelle, die sich ausgehend von der Messfläche, insbesondere von dem Bereich der Messfläche, in dem der Anregungsstrahl diese durchsetzt, durch den Messkörper ausbreitet, zeitlich nacheinander, insbesondere in zeitlich gegeneinander verschobenen Zeitabschnitten oder mit einem zeitlichen Abstand passiert werden.

Eine Druck- und/oder Wärmewelle, die sich, ausgehend von dem Bereich der Messfläche, in dem der Anregungsstrahl diese durchsetzt, durch den Messkörper ausbreitet, erreicht dann zunächst einen ersten der Messabschnitte und ändert dort während ihrer Durchlaufzeit vorübergehend den Brechungsindex. In dem Zeitintervall, während dieser geänderte Brechungsindex wirkt, wird eine erste Phasenverschiebung gegenüber dem Detektionslicht erzeugt, das den zweiten Messabschnitt durchläuft (beide Messabschnitte werden parallel von dem Detektionslicht durchlaufen). Diese Phasenverschiebung kann durch die oben beschriebene Intensitätsmessung des Detektionslichts nachgewiesen werden. Danach erreicht die Welle den zweiten Messabschnitt und entfaltet dort ihre Wirkung, indem auch dort der Brechungsindex für ein Zeitintervall geändert wird. Überschneiden sich die beiden Zeitintervalle, so neutralisieren sich die Phasenverschiebungen für den Zeitbereich der Überschneidung wenigstens teilweise. Danach, wenn nur im zweiten Messabschnitt die Phasenverschiebung stattfindet, tritt wieder die Wirkung der Intensitätsänderung des Detektionslichts ein. Dieser zeitliche Verlauf kann durch eine Auswerteeinrichtung erfasst werden und hieraus kann die Änderung des Brechungsindex in dem ersten Messabschnitt und in dem zweiten Messabschnitt ermittelt werden. Der ermittelten Änderung des Brechungsindex kann eine Temperatur- und/oder Druckänderung zugeordnet werden, die ein Maß für die Absorptionsstärke des Anregungsstrahls in dem zu analysierenden Stoff darstellt. Dabei verlaufen die genannten Messabschnitte mit ihren Lichtleiter-Längsachsen vorteilhaft quer, insbesondere senkrecht zur Ausbreitungsrichtung der Druck- und/oder Temperaturwelle im Messkörper und weiter insbesondere von dem Bereich der Messfläche, in dem der Anregungsstrahl diese durchsetzt, aus gesehen hintereinander.

Es ist weiter vorteilhaft eine Auswerteeinrichtung vorgesehen, die aus der Intensitätsänderung des Detektionslichts in der Lichtwellenleiterstruktur einen Änderungsbetrag die Phasenverschiebung des Detektionslichts in einem Messabschnitt und aus dieser die Änderung des Brechungsindex ermittelt. Aus dieser Änderung des Brechungsindex kann die Druck- und/oder Temperaturänderung in den Messabschnitten und aus dieser die Absorptionsstärke des Anregungsstrahls in dem zu analysierenden Stoff ermittelt werden.

Beschrieben wird auch ein Sensor, der beispielsweise für eine Einrichtung der oben erläuterten Art verwendbar ist, mit einem Messkörper, der eine Messfläche aufweist und im Bereich der Messfläche zur Messung einer Temperatur- und/oder Druckwelle wenigstens teilweise mit einem Stoff zu koppeln, insbesondere in Kontakt zu bringen ist, und mit einer wenigstens teilweise in den Messkörper integrierten oder mit diesem verbundenen Detektionsvorrichtung, die folgendes umfasst:
- eine Quelle für kohärentes Detektionslicht sowie
- eine mit der Quelle für das Detektionslicht verbindbare oder verbundene und das Detektionslicht führende erste Lichtwellenleiterstruktur, deren Brechungsindex wenigstens abschnittsweise von der Temperatur und/oder dem Druck abhängig ist,
- wenigstens einen Abschnitt , in dem die Lichtintensität von einer Phasenverschiebung von Detektionslicht in wenigstens einem Teil der ersten Lichtwellenleiterstruktur durch eine Temperatur- oder Druckänderung abhängt, wobei die erste Lichtwellenleiterstruktur eine interferometrische Einrichtung, insbesondere ein Interferometer und/oder einen Lichtwellenleiter-Resonanzring oder ein anderes Lichtwellenleiter-Resonanzelement aufweist, sowie
- eine Messeinrichtung zur Erfassung der Lichtintensität in oder an der interferometrischen Einrichtung.

Sämtliche in dieser Anmeldung erläuterten Merkmale zur Ausgestaltung des Temperatursensors der erfindungsgemäßen Analyseeinrichtung können auch zur Implementierung eines Sensors losgelöst von der Analyseeinrichtung für andere Zwecke herangezogen werden, insbesondere alle beschriebenen Anordnungen, Ausgestaltungen, Materialwahlen, Herstellungsarten und Formgebungen eines integrierten optischen Resonanzrings oder des Messarms und des Referenzarms eines Interferometers.

Mit diesem Sensor sind Temperaturänderungen oder auch Druckwellen messbar, welche mittels Brechungsindexänderungen erfasst werden können. Außer für die oben erläuterten Zwecke kann somit der Sensor beispielsweise auch für Erschütterungsmessungen, beispielsweise seismische Messungen oder mechanische Impulsmessungen verwendet werden. Der Sensor ist hierbei durch seine kurze Ansprechzeit für Messungen qualifiziert, bei denen andere Sensoren wie MEMS- Sensoren wegen ihrer Trägheit nicht einsetzbar sind.

Die Erfindung bezieht sich außer auf eine Einrichtung der oben erläuterten Art auch auf ein Verfahren zum Betrieb einer solchen Einrichtung, wobei vorgesehen ist, dass ein modulierter Anregungsstrahl, insbesondere durch den Messkörper hindurch, auf den zu analysierenden Stoff gerichtet wird und dass durch die Detektionsvorrichtung ein Lichtintensitätsverlauf oder eine periodische Lichtintensitätsänderung erfasst wird, wobei diese für eine Mehrzahl von Wellenlängen des Anregungsstrahls durch die Messung der Lichtintensitätsänderung in der ersten Lichtwellenleiterstruktur oder durch die Messung der Lichtintensität von aus der ersten Lichtwellenleiter ausgekoppeltem Licht erfasst werden und aus den erfassten Daten ein Absorptionsspektrum des zu analysierenden Stoffs gewonnen wird.

Bei einem solchen Verfahren kann zudem vorgesehen sein, dass die Messung für verschiedene Modulationsfrequenzen des Anregungsstrahls durchgeführt wird und dass aus der Verknüpfung von gewonnenen Absorptionsspektren ein korrigiertes Absorptionsspektrum ermittelt wird. Damit lässt sich eine Tiefenprofilierung der Konzentration einer analysierten Substanz in dem untersuchten Stoff ermitteln oder es lassen sich störende Effekte aus bestimmten Tiefenbereichen durch mathematische Verknüpfung reduzieren oder eliminieren.

Beschrieben wird auch ein Verfahren zum Analysieren eines Stoffes, insbesondere unter Verwendung einer Einrichtung der oben erläuterten Art, wobei bei dem Verfahren
- mit einer Anregungssendeeinrichtung mindestens ein intensitätsmodulierter elektromagnetischer Anregungsstrahl mit zumindest einer Anregungswellenlänge erzeugt wird, die Anregungssendeeinrichtung den mindestens einen elektromagnetischen Anregungsstrahl in ein Stoffvolumen einstrahlt, das unterhalb der Oberfläche des Stoffs liegt,
- mit einer Detektionsvorrichtung ein Reaktionssignal in Form einer Lichtintensität in der ersten Lichtwellenleiterstruktur detektiert wird, und
- anhand des detektierten Reaktionssignals der Stoff analysiert wird, wobei Reaktionssignale, insbesondere zeitliche Reaktionssignalverläufe für verschiedene Wellenlängen des Anregungsstrahls ermittelt werden und aus dem Abklingverhalten der Reaktionssignale jeweils nach dem Ende einer Modulationsphase, in der der Anregungsstrahl eine hohe Intensität aufweist, eine Information über das Tiefenprofil unter der Oberfläche des zu analysierenden Stoffes gewonnen wird, in dem der Anregungsstrahl absorbiert und die Wärme- und/oder Druckwelle erzeugt wird.

Es kann auch vorgesehen sein, dass
- nacheinander unter Verwendung verschiedener Modulationsfrequenzen der Anregungssendeeinrichtung mehrere Reaktionssignalverläufe ermittelt werden und
- mehrere Reaktionssignalverläufe zu verschiedenen Modulationsfrequenzen miteinander verknüpft werden und wobei
- daraus insbesondere eine für einen Tiefenbereich unter der Stoffoberfläche spezifische Information gewonnen wird.

Mittels der Detektionsvorrichtung kann, insbesondere in dem Fall, dass eine Druckänderung erfasst wird, auch ein Reaktionssignal in Form einer Schallwelle nachgewiesen werden, die durch die Absorption des Anregungsstrahls in dem zu analysierenden Stoff erzeugt wird und mit bekannter Geschwindigkeit (in menschlichem Gewebe ca. 1500m/s) zur Messfläche und zum Detektionsbereich wandert. Mittels einer Auswerteeinrichtung, die mit einer Modulationseinrichtung für den Anregungsstrahl verbunden ist, kann wegen der guten Zeitauflösung der Messung der Reaktionssignale eine Phasenverschiebung zwischen der Modulation des Anregungsstrahls und dem Reaktionssignal gemessen und dadurch die Tiefe im Gewebe, in der die Absorption stattgefunden hat, ermittelt werden. Da die Signale häufig eine Überlagerung verschiedener Reaktionssignale aus verschiedenen Gewebeschichten sind, kann eine Interpretation der Signale durch eine Modellbildung mit einer Mehrzahl von in verschiedenen Tiefen des Stoffes verteilten Absorptionsstellen und diesen zugeordneten Absorptionsstärken sowie Laufzeiten bis zur Stoffoberfläche stattfinden, wobei die Absorptionsstärken an den zeitlichen Reaktionssignalverlauf angefittet werden und somit der Reaktionssignalverlauf rekonstruiert werden kann. Daraus können die Absorptionsstärken und damit die lokalen Konzentrationen der in dem Stoff nachzuweisenden Substanz bestimmt werden.

Alternativ oder zusätzlich können auch verschiedene Messungen mit verschiedenen Modulationsfrequenzen durchgeführt und die Reaktionssignale zu verschiedenen Modulationsfrequenzen miteinander verknüpft werden, um insbesondere Signale aus oberen Gewebeschichten herauszurechnen und zu eliminieren, da diese durch Verschmutzung und abgestorbene Hautzellen besonders fehleranfällig sind.

Die oben genannte Einrichtung kann auch vorteilhaft kombiniert werden
- mit wenigstens einer weiteren Detektionsvorrichtung, die der Messfläche benachbart angeordnet ist und/oder an diese unmittelbar angrenzt, wobei die weitere Detektionsvorrichtung eine Kontakteinrichtung mit wenigstens 2 Elektroden zur Erfassung von piezoelektrischen Signalen aufweist, die einander auf verschiedenen Seiten eines Detektionsbereiches gegenüberliegen. Im Detektionsbereich ist dabei ein Material angeordnet, das in Abhängigkeit von Temperatur- und /oder Druckänderungen, insbesondere aufgrund eines Piezoelektrischen Effekts, seinen elektrischen Widerstand ändert oder ein elektrisches Signal erzeugt.

Mittels dieser weiteren Detektionsvorrichtung kann beispielsweise in alternativer Weise eine Temperatur oder ein Druck gemessen werden, wobei diese Messung als Referenzmessung für eine Umgebungstemperatur oder einen Umgebungsdruck verwendet werden kann oder auch zur Messung der aus dem zu analysierenden Stoff ausgestrahlten Wärme- und/oder Temperaturwelle, um die Messwerte, die durch die Detektionsvorrichtung gewonnen werden, mit Messwerten der weiteren Detektionsvorrichtung zu verknüpfen.

Im Folgenden wird die Einrichtung anhand von Figuren einer Zeichnung gezeigt und nachfolgend erläutert.

Dabei zeigt
- Figur 1: schematisch eine Seitenansicht eines Messkörpers mit einer Lasereinrichtung und einer Detektionsvorrichtung,
- Figur 2: eine Seitenansicht eines Messkörpers,
- Figur 3: eine Seitenansicht eines weiteren Messkörpers,
- Figur 4: eine Draufsicht auf eine erste Lichtwellenleiterstruktur an einem Messkörper,
- Figur 5: eine Draufsicht auf eine weitere Implementierung einer ersten Lichtwellenleiterstruktur an einem Messkörper,
- Figur 6: einen Querschnitt durch ein Substrat mit integrierten Lichtwellenleitern,
- Figuren 6a-6i: verschiedene Ausführungsformen eines oder mehrerer Substrate mit einer interferometrischen Einrichtung, wobei die Schraffur des Messkörpers bei einigen Darstellungen eingezeichnet und bei anderen Darstellungen der Übersichtlichkeit halber weggelassen ist,
- Figur 6k: eine Ausführungsform mit einer interferometrischen Einrichtung, bei der der zeitliche Verlauf der Phasenverschiebung/Brechzahländerung in Abhängigkeit vom Passieren der verschiedenen Messabschnitte durch eine Druck- und/oder Wärmewelle erfasst werden kann,
- Figur 6l: den zeitlichen Verlauf der Phasenverschiebung des Detektionslichtes in den Messabschnitten während des Durchlaufes einer Druck- und/oder Wärmewelle,
- Figur 6m: einen Verlauf eines Anregungsstrahls an einer äußeren Grenzfläche eines Messkörpers vorbei in den Stoff sowie die Position einer interferometrischen Einrichtung,
- Figur 6n: einen Messkörper mit einem akustischen Koppelelement zur Kopplung an den zu analysierenden Stoff,
- Figur 7: eine Querschnittsansicht durch ein weiteres Substrat mit integrierten Lichtwellenleitern,
- Figur 8: einen Querschnitt durch ein Substrat mit aufgeklebten Lichtwellenleitern,
- Figur 9: eine Querschnittsansicht eines Substrats mit einer durchgehenden Öffnung für einen Anregungsstrahl,
- Figur 10: eine Querschnittsansicht eines Substrats mit einer weiteren durchgehenden Öffnung für einen Anregungsstrahl,
- Figur 11: eine Querschnittsansicht eines Substrats mit einer zweiten Lichtwellenleiterstruktur für einen Anregungsstrahl,
- Figur 12: eine Querschnittsansicht eines Substrats mit einer weiteren Implementierung einer zweiten Lichtwellenleiterstruktur für einen Anregungsstrahl,
- Figur 13: eine schematische Übersicht über die Einrichtung zur Analyse eines Stoffs mit einer Verarbeitungseinrichtung für Messergebnisse sowie Ausgabeeinrichtungen für Signale,
- Fig. 14 bis 16: eine Anordnung mit einem Substrat, mit dem die Anregungslichtquelle und die Detektionslichtquelle sowie ein Detektor verbunden sind und in die ein weiteres Substrat mit integrierten optischen Elementen einsetzbar ist,
- Figur 17:: einen Querschnitt eines Messkörpers mit einer ersten integrierten Linse und mit einem auf die Messfläche aufgelegten Finger,
- Figur 18: einen Querschnitt eines Messkörpers mit einer zweiten integrierten Linse,
- Figur 19: einen Querschnitt eines Messkörpers mit einer dritten integrierten Linse,
- Figur 20: einen Querschnitt eines Messkörpers mit einer ersten integrierten Linse und einem Anregungsstrahl,
- Figur 21: einen Querschnitt eines Messkörpers mit einer zweiten integrierten Linse und einem Anregungsstrahl,
- Figur 22: einen Querschnitt eines Messkörpers mit einer dritten integrierten Linse und einem Anregungsstrahl, sowie
- Figuren 23, 24, 25: mehrere Anordnungen mit einem Messkörper und einer Anregungslichtquelle in Form einer Laser- Lichtquelle oder Anregungsstrahlquelle, insbesondere einer Lasereinrichtung, wobei der Anregungslichtstrahl mittels eines in ein Substrat des Messkörpers integrierten Lichtwellenleiters durch den Messkörper zur Messfläche geführt ist.

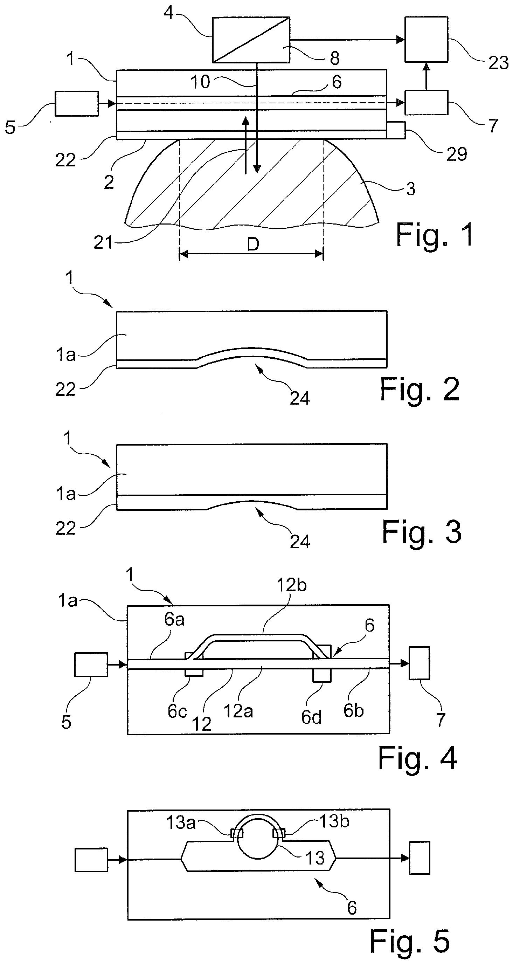

Die Figur 1 zeigt in einer Querschnittsansicht einen Messkörper 1, auf dessen inneren Aufbau in dieser Figur nicht näher eingegangen wird. Innerhalb des Messkörpers 1 ist eine erste Lichtwellenleiterstruktur 6 schematisch angezeigt, in die durch eine Detektionslichtquelle 5 kohärentes Detektionslicht eingestrahlt wird. Mittels einer Messeinrichtung 7 wird eine Lichtintensität in der ersten Lichtwellenleiterstruktur 6 nachgewiesen, die von dem auf die Lichtwellenleiterstruktur 6 einwirkenden Druck oder der Temperatur abhängig ist.

Die Detektionslichtquelle 5 kann als Laser oder Laserdiode ausgebildet und an dem Messkörper 1 angeordnet oder befestigt sein. Die Detektionslichtquelle 5 kann mit der ersten Lichtwellenleiterstruktur 6 auch mittels eines Glasfaserlichtwellenleiters in flexibler Weise verbunden sein. Zudem kann die Detektionslichtquelle 5 als Halbleiterelement in ein Substrat (hier nicht gezeigt) innerhalb des Messkörpers 1 integriert und dort mit einer ersten Lichtwellenleiterstruktur verbunden sein.

Auch die Messeinrichtung 7 kann an die erste Lichtwellenleiterstruktur 6 direkt mittels eines Kopplers angekoppelt oder mit dieser mittels eines integrierten Lichtwellenleiters oder eines flexiblen Glasfaserlichtwellenleiters (hier nicht gezeigt) verbunden sein. Die Messeinrichtung 7 kann jedoch auch in den Messkörper integriert und auf einem Substrat des Messkörpers 1 als Halbleiterelement realisiert sein. Die Messeinrichtung 7 kann beispielsweise als lichtempfindliches Halbleiterelement, beispielsweise als Photodiode ausgebildet sein.

Zusätzlich zu den genannten Komponenten kann auch eine Temperaturmesseinrichtung zur Messung der absoluten Temperatur des Messkörpers 1 vorgesehen sein, um eine über längere Zeitintervalle, beispielsweise eine Zehntelsekunde, eine halbe Sekunde, eine oder mehrere Sekunden, gemessene Durchschnittstemperatur je nach der Zeitkonstante der übrigen Sensoren bei der Auswertung der Messungen zu berücksichtigen. Dadurch kann beispielsweise die Temperaturabhängigkeit einer Photodiode oder eines anderen Halbleiter-Lichtsensors korrigiert werden. Dies kann beispielsweise bei der Auswertung der durch die Messeinrichtung 7 gemessenen Lichtintensität sinnvoll sein, die durch eine Temperaturkorrektur verbessert werden kann. Alternativ dazu kann auch eine Temperaturstabilisierungseinrichtung 29 vorgesehen sein, die ein Heiz- oder ein Kühlelement enthält und die den Messkörper 1 auf einer konstanten Temperatur hält. Diese Temperatur kann beispielsweise einer mittleren Umgebungstemperatur entsprechen, die festgelegt werden kann, beispielsweise auf 20°C, sie kann jedoch auch einer mittleren Körpertemperatur eines Patienten entsprechen, dessen Körpergewebe oder Körperflüssigkeit vermessen werden soll und die damit beispielweise bei etwa 37°C oder bei 30°C (freiliegende Hautoberfläche) liegen kann.

In der Figur 1 ist eine Lasereinrichtung 4 dargestellt, die als Quantenkaskadenlaser oder als Laserarray ausgebildet sein kann. Der Quantenkaskadenlaser kann so ausgestaltet sein, dass er wenigstens teilweise bezüglich seiner Wellenlänge durchstimmbar, insbesondere im Infrarotbereich, weiter insbesondere im mittleren Infrarotbereich durchstimmbar, ist. Ist die Lasereinrichtung 4 als Laserarray aufgebaut, so können einzelne Laserelemente des Arrays durchstimmbar, einstellbar oder auf bestimmte Wellenlängen festgelegt sein. Die Wellenlängen der einzelnen Laserelemente können beispielsweise so festgelegt werden, dass sie den Wellenlängen von Absorptionsmaxima einer nachzuweisenden Substanz in dem zu analysierenden Stoff entsprechen, also beispielsweise den Absorptionsmaxima von Glukose. Die Wellenlänge der Anregungsstrahlen kann für die hier beispielhaft erläuterte Blutzuckermessung - vorzugsweise derart gewählt werden, dass die Anregungsstrahlen von Glukose bzw. Blutzucker signifikant absorbiert werden. Zur Messung von Glukose bzw. Blutzucker sind folgende glucoserelevante Infrarotwellenlängen besonders gut geeignet (Vakuumwellenlängen) und können einzeln oder gruppenweise gleichzeitig oder nacheinander als feste Wellenlängen für eine Messung der Reaktionssignale eingestellt werden: 8,1 µm, 8,3 µm, 8,5 µm, 8,8 µm, 9,2 µm, 9,4 µm und 9,7 µm. Zudem können glucosetolerante Wellenlängen verwendet werden, die von Glucose nicht absorbiert werden, um andere vorhanden Stoffe zu ermitteln und deren Einfluss auf die Messung auszuschließen.

Da mit der Einrichtung auch andere beispielsweise biologische oder chemische Stoffe nachweisbar und analysierbar sind, kommen hier jedoch auch die Absorptionsmaxima der nachzuweisenden Substanzen in Frage. Als Zahl der Sendeelemente eines Laserarrays kommt beispielsweise eine Zahl von 10 bis 20 oder eine Zahl von 10 bis 30 Elementen oder auch eine Zahl größer als 30 Sendeelemente in Frage.

Die Lasereinrichtung 4, die auch Anregungsstrahlerzeugungseinrichtung oder Anregungsstrahlsendeeinrichtung genannt werden kann, weist eine Modulationseinrichtung 8 auf, die einen modulierten Laserstrahl erzeugt. Die Modulationseinrichtung 8 kann in diesem Fall beispielsweise in der Ansteuerung der Lasereinrichtung 4 angeordnet sein. Die Modulationsfrequenz kann beispielsweise bei einer Frequenz zwischen 100 Hz und einigen Megahertz oder sogar einigen hundert Megahertz liegen. Wichtig ist dabei, dass die erste Lichtwellenleiterstruktur 6 eine passende Reaktionszeit hat und auf entsprechend der Modulationsfrequenz eintreffende ebenfalls intensitätsmodulierte Druck- oder Wärmewellen reagieren kann. Das ist bei Verwendung der weiter unten näher erläuterten interferometrischen Detektionsvorrichtungen der Fall.

Licht der Lasereinrichtung 4 tritt als Anregungsstrahl 10 durch eine Messfläche 2, die als untere Oberfläche des Messkörpers 1 dargestellt ist, in den Bereich aus, der mit D bezeichnet ist und in dem der zu analysierende Stoff 3 mit der Messfläche 2 in Kontakt tritt. Nach Absorption des Anregungslichtstrahls 10 in dem Stoff 3 wird aus dem Stoff eine Temperatur- und/oder Druckwelle 21 zu dem Messkörper 1 geleitet und trifft auf die erste Lichtwellenleiterstruktur 6. Die Temperatur- und/oder Druckänderung ruft dort eine Intensitätsänderung des Detektionslichts hervor, die mittels der Messeinrichtung 7 erfasst und an eine Verarbeitungseinrichtung 23 weitergegeben wird. Die Verarbeitungseinrichtung 23 kann über einen Lock-in-Verstärker verfügen, der die Signale im Gleichtakt mit der Modulation des Anregungsstrahls 10 verstärkt.

Optional kann der Messkörper 1 im Bereich der Messfläche 2 mit einer Beschichtung 22 versehen sein, auf die der zu analysierende Stoff 3 unmittelbar aufgebracht werden kann. Dies kann sinnvoll sein, um ein in dem Messkörper 1 vorgesehenes Substratmaterial zu schützen oder die mechanische und/oder thermische Ankopplung des Stoffs 3 an die erste Lichtwellenleiterstruktur 6 zu begünstigen. Das Material der Beschichtung 22 sollte derart gestaltet sein, dass es Druck- und Wärmewellen gut weiterleitet. Zudem kann es transparent für den Anregungsstrahl 10 gewählt sein. Eine Abdeckschicht 22, die grundsätzlich auch zwischen der ersten Lichtwellenleiterstruktur 6 und einem zu analysierenden Stoff, also beispielsweise auf einer Oberfläche der ersten Lichtwellenleiterstruktur 6 vorgesehen sein kann, kann auch dazu dienen, eine unmittelbare Wechselwirkung der Strahlung innerhalb der ersten Lichtwellenleiterstruktur 6 oder zumindest die Wechselwirkung eines evaneszenten Teils dieser Strahlung außerhalb der eigentlichen Lichtwellenleiterstruktur 6 mit einem auf die Messfläche 2 aufgebrachten Stoff zu verhindern, da ein solcher Kontakt eine Rückwirkung auf die Strahlung in der ersten Lichtwellenleiterstruktur 6 zur Folge haben könnte.

Es kann auch eine akustische Kopplung des Messkörpers an den zu analysierenden Stoff vorgesehen sein, bei der der Messkörper mittels eines zwischen diesem und dem Stoff eingefügten Mediums die in dem Stoff erzeugten Wellen aufnimmt. Das Medium kann als Fluid, also gasförmig oder flüssig ausgebildet sein, so dass zwischen dem Messkörper und dem Stoff ein Abstand, beispielsweise in Form eines Hohlraums oder einer Ausnehmung im Messkörper vorgesehen sein kann. Die Öffnung des Hohlraums kann dann an den Stoff angelegt werden, so dass die Welle durch den Hohlraum in den Messkörper gelangen kann. Die Wand des Hohlraums, also die Außenfläche des Messkörpers, kann mit einem Material beschichtet sein, dass eine gute akustische Ankopplung, also eine Impedanzanpassung bewirkt.

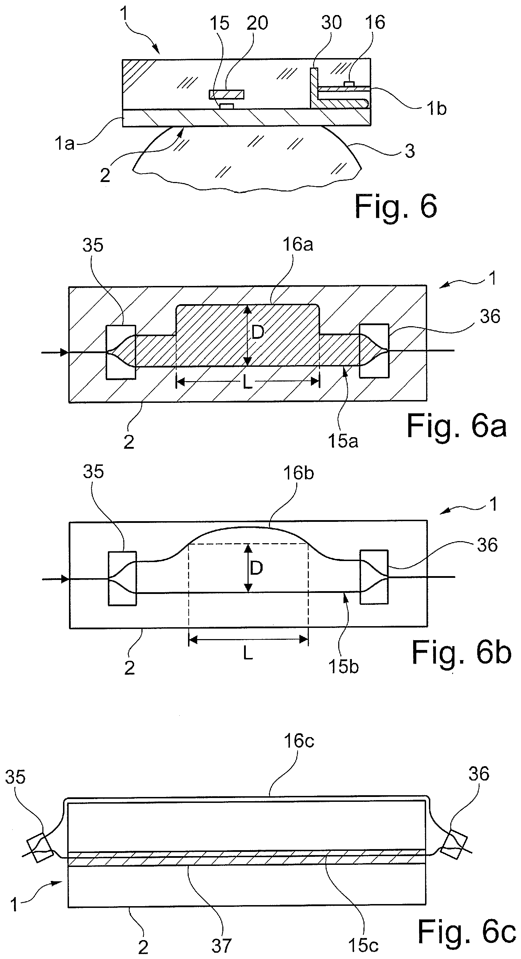

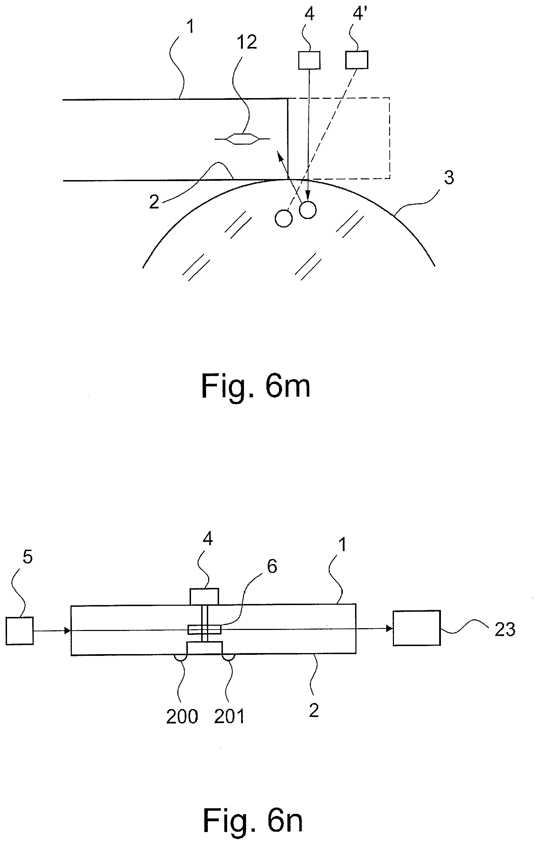

Eine derartige akustische Ankopplung wird weiter unten noch detaillierter anhand der Figur 6n gezeigt und erläutert.

In der Figur 2 ist in einer Seitenansicht dargestellt, dass der Messkörper 1 eine Mulde 24 ausbilden kann, die mit der Beschichtung 22 bedeckt ist. Die Mulde 24 ist dazu vorgesehen, in diesem Bereich den zu analysierenden Stoff 3 an die Messfläche 2 anzulegen. Hierdurch ist den Nutzer der Einrichtung eine Orientierung gegeben. Zudem kann durch die Mulde bei Anlegen eines Körperteils, beispielsweise einer Fingerbeere an die Messfläche 2, eine mechanische Stabilisierung erfolgen.

In der Figur 3 ist als alternative Ausgestaltung gezeigt, dass die Mulde 24 ausschließlich durch einen Bereich gebildet ist, in dem die Beschichtung 22 bezüglich ihrer Dicke verringert ist. Dadurch kann beispielsweise ein innerhalb des Messkörpers 1 vorgesehenes Substrat 1a als ebener planparalleler Körper unbearbeitet eingesetzt werden.

In der Figur 4 ist eine Draufsicht auf ein Substrat 1a dargestellt, das Teil eines Messkörpers 1 sein kann. Das Substrat 1a ist als flacher planparalleler Körper beispielsweise aus Silizium ausgebildet, insbesondere als Wafer, der dünner als 1 mm ausgebildet sein kann. Als Substrat kann jedoch auch eine Sandwichstruktur vorgesehen sein, die mehrere Waferschichten oder einen dickeren Wafer mit einer oder mehreren Ausnehmungen, insbesondere ausgeätzten Bereichen, aufweist. Auf oder in das Substrat 1a ist eine Lichtwellenleiterstruktur 6 in Form eines Interferometers aufgebracht. Dies kann beispielsweise dadurch geschehen, dass zunächst der Siliziumwafer mit einer Siliziumoxidschicht abgedeckt und auf diese Silizium-lichtwellenleiter aufgebracht werden. Diese können darauf ihrerseits mit einer Siliziumoxidschicht abgedeckt werden.

Das Substrat 1a kann dann als Ganzes auf einer oder beiden Seiten mit einer Schutz- oder Funktionsschicht abgedeckt werden, die beispielsweise ebenfalls aus Silizium oder auch aus einem Polymer oder Glas bestehen kann.

Das dargestellte Interferometer 12 ist als Mach-Zehnder-Interferometer ausgebildet und weist einen Messarm 12a und einen Referenzarm 12b auf. Das durch die Detektionslichtquelle 5 erzeugte Detektionslicht wird durch einen Eingangslichtwellenleiter 6a der ersten Lichtwellenleiterstruktur 6 zu einem Strahlteiler 6c geleitet, wo das Licht in zwei Teillichtstrahlen durch den Mess- bzw den Referenzarm 12a, 12b aufgeteilt wird. Der Referenzarm 12b kann einen Mindestabstand von wenigstens 1 mm oder wenigstens 2 mm oder wenigstens 5 mm oder wenigstens 8 mm von dem Messarm 12a aufweisen, um eine Beeinflussung des Referenzarms 12b durch eine Wirkung der eintreffenden Temperatur und/oder Druckwelle möglichst weitgehend auszuschließen oder zu reduzieren. Der Messkörper 1 wird dann derart relativ zu dem Anregungslichtstrahl 10 positioniert, dass eine aus dem zu analysierenden Stoff ausgesendete Temperatur und/oder Druckwelle weit überwiegend den Messarm 12a des Interferometers erreicht und dort den Brechungsindex des Lichtwellenleiters beeinflusst.

Die beiden Arme des Interferometers können beispielsweise in einer Ebene liegen, die parallel zur Messfläche liegt, jedoch auch in einer Ebene, die senkrecht zur Messfläche orientiert ist.

Im Ergebnis wird eine Phasenverschiebung der in den verschiedenen Armen des Interferometers laufenden Lichtstrahlen erreicht, die bei der Kopplung der Lichtstrahlen in dem zweiten Koppler 6d je nach Phasenlage zu einer Auslöschung oder Teilauslöschung des Detektionslichts führt. Die Intensität des Detektionslichts wird dann in Ausleitlichtwellenleiter 6b der ersten Lichtwellenleiterstruktur 6 oder an dessen Ende oder einer Auskoppelstelle durch die Messeinrichtung 7 erfasst. Das Detektionslicht kann beispielsweise Wellenlängen im sichtbaren Bereich oder auch im Infrarotbereich umfassen.

Alternativ kann auch anstelle eines Interferometers ein Lichtwellenleiter- Resonanzelement, beispielsweise ein Ringresonator oder ein Plattenresonator, mit einem Einkoppelelement für Detektionslicht und einem Auskoppelelement als Sensor für Druck- und/oder Temperaturänderungen eingesetzt werden.

In der Figur 5 ist als Variante eines interferometrischen Aggregats ein Interferometer gezeigt, das mit einem Lichtwellenleiterresonanzring 13 kombiniert ist. Dies ist dadurch realisiert, dass der Messarm des Interferometers an zwei Koppelstellen 13a, 13b mit dem Resonanzring 13 gekoppelt ist. Durch die Integration eines Resonanzrings 13 in einen Arm eines Interferometers kann eine deutlich höhere Temperaturempfindlichkeit der Anordnung erzielt werden.

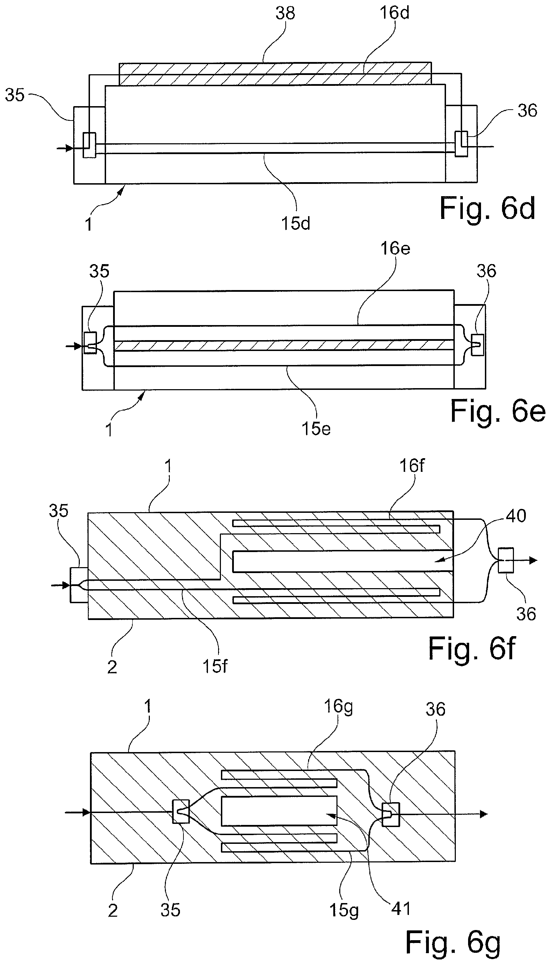

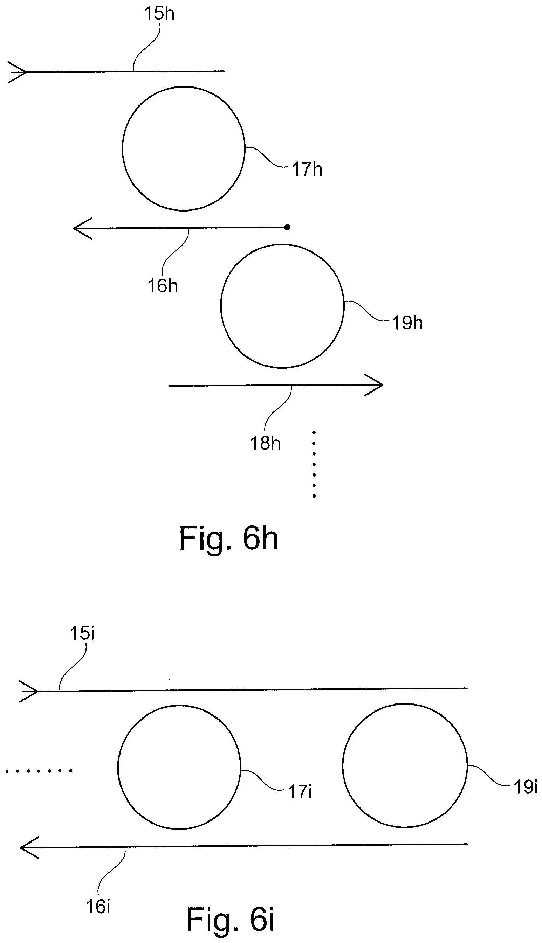

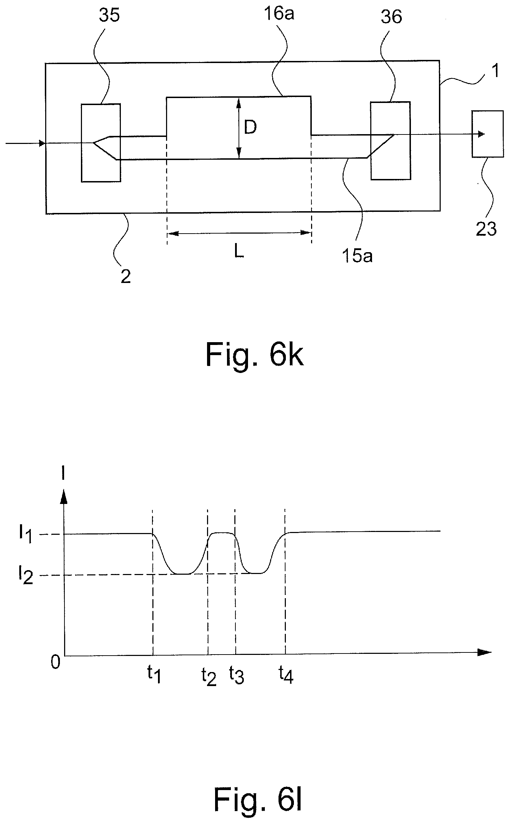

In der Figur 6 ist ein Querschnitt durch einen Messkörper 1 mit einem Substrat 1a gezeigt. Auf dem Substrat 1a ist ein erster Lichtwellenleiter 15 einer ersten Lichtwellenleiterstruktur angeordnet. Der erste Lichtwellenleiter 15 kann auf dem Substrat 1a integriert sein. Von der Messfläche 2 aus gesehen hinter dem Lichtwellenleiter 15 ist eine Wärmesenke 20 in Form eines Körpers vorgesehen, der oberhalb des Lichtwellenleiters 15 parallel zu diesem läuft und in das Material des Messkörpers eingelassen, beispielsweise eingegossen, ist. Die Wärmesenke 20 kann auch unmittelbar auf dem Lichtwellenleiter 15 aufliegen. Das Material der Wärmesenke 20 weist eine höhere spezifische Wärmeleitfähigkeit und/oder eine höhere spezifische Wärmekapazität auf als das Material des Lichtwellenleiters 15 und/oder als das Material des Substrats 1a und/oder als ein Material, mit dem das Substrat 1a bedeckt ist.

Der erste Lichtwellenleiter 15 stellt beispielsweise einen Messarm eines Interferometers dar. Der entsprechende Referenzarm ist als zweiter Lichtwellenleiter 16 ausgebildet und auf einem weiteren Substrat 1b integriert, das entweder mit dem Substrat 1a zusammenhängend hergestellt sein oder mit diesem in einem gemeinsamen Messkörper 1 gekoppelt und vergossen werden kann. Zwischen der Messfläche 2, insbesondere zwischen dem Substrat 1a, und den zweiten Lichtwellenleiter 16 ist eine Wärmebarriere 30 angeordnet, die sich wenigstens abschnittsweise parallel zu dem zweiten Lichtwellenleiter 16 zwischen diesem und der Messfläche erstreckt und diesen von der Wirkung einer Druck- und/oder Temperaturwelle abschirmt, die durch die Messfläche 2 hindurchtritt. Alternativ oder zusätzlich zu der Wärmebarriere 30 kann der Lichtwellenleiter 16 durch einen Gasspalt von dem Bereich der Messfläche 2 abgeschirmt sein. Ein solcher Gasspalt kann beispielsweise durch Ätzen oder ein anderes abtragendes Verfahren in das Substrat 1a eingebracht werden oder er kann in einem Vergusswerkstoff vorgesehen sein, mit dem das Substrat 1a zu dem Messkörper 1 vergossen wird. Die Wärmebarriere 30 kann auch in Form eines Körpers als Barriere gegen eine Druckwelle ausgebildet sein und zu diesem Zweck eine Plastizität oder Elastizität aufweisen, die höher ist als diejenige des Materials des Messkörpers 1, das den Lichtwellenleiter 16 unmittelbar umgibt. In vielen Fällen und aufgrund der geringen Baugröße der interferometrischen Elemente wird es sinnvoll sein, die Wärmebarriere durch geätzte Gräben in einem Substrat, beispielsweise in dem Substrat 1a oder dem Substrat 1b, zu realisieren. Die Wärmebarriere weist beispielsweise eine Leitfähigkeit für Druck- oder Wärmewellen auf, die deutlich geringer ist als die eines Vergussmaterials des Messkörpers oder des Substrats 1a, 1b.

Die Figuren 6a bis 6g zeigen verschiedene Ausführungsformen einer interferometrischen Einrichtung, bei denen jeweils der Messarm und der Referenzarm so ausgebildet sind, dass die Auswirkung einer Temperatur- und/oder Druckwelle auf den Referenzarm bezüglich einer Veränderung des Brechungsindex geringer ist als die Auswirkung auf den Messarm. Dies wird in einigen Fällen dadurch erreicht, dass der Referenzarm in größerer Entfernung von der Messfläche 2 positioniert wird als der Messarm. In manchen Fällen ist ein Hindernis oder eine Barriere zwischen dem Referenzarm und der Messfläche 2 vorgesehen. In weiteren Fällen wird der Referenzarm von dem Substrat entkoppelt oder beabstandet, während der Messarm mit dem Substrat wärmeleitend und/oder in starrer mechanischer Kopplung verbunden ist.

Die Figur 6a zeigt einen Messarm in Form eines Lichtwellenleiters 15a und einen Referenzarm in Form eines Lichtwellenleiters 16a. Ein Strahlteiler bzw. Splitter ist mit 35 bezeichnet, während ein Koppler, in dem die Strahlen des Messarms und des Referenzarms wieder zusammengeführt sind, mit 36 bezeichnet ist. Der Referenzarm ist in einem mittleren Bereich des Messkörpers 1 von dem Messarm auf einer Länge L in einem Abstand D geführt. Der Referenzarm ist auf der der Messfläche 2 abgewandten Seite des Messarms angeordnet und somit von der Messfläche 2 um den Betrag D weiter entfernt als der Messarm.

Die Figur 6b zeigt einen Messarm in Form eines Lichtwellenleiters 15b und einen Referenzarm in Form eines Lichtwellenleiters 16b. Auch hier, wie auch bei den folgenden Figuren, ist der Strahlteiler, der das Detektionslicht auf den Messarm 15b und den Referenzarm 16b verteilt, mit 35 und der Koppler mit 36 bezeichnet. Splitter und Koppler können jeweils als separates optisches Element oder als in das Substrat des Messkörpers 1 integriertes Element ausgebildet sein.

Der Referenzarm 16b ist in einem mittleren Bereich des Messkörpers 1 von dem Messarm 15b in einem Abstand von wenigstens dem Betrag D geführt.

Zwischen dem Messarm und dem Referenzarm kann jeweils auch eine hier nicht eingezeichnete Barriere vorgesehen sein, die Wärme- und/oder Druckwellen vom Referenzarm fernhält.

Der Messarm kann auch eine Länge aufweisen, die größer ist als die Länge des Referenzarms, dadurch, dass der Messarm wenigstens abschnittsweise in Schleifen verläuft und/oder spiralförmig oder mäanderförmig verläuft. Es kann aber zudem auch vorgesehen sein, dass der Referenzarm wenigstens abschnittsweise in Schleifen verläuft und/oder spiralförmig oder mäanderförmig verläuft. Schleifen, Spiralen oder Mäanderförmige Abschnitte des Messarms und/oder des Referenzarms können grundsätzlich in einer Ebene parallel zur Messfläche 2 verlaufen, jedoch auch in einer Ebene, die senkrecht zur Messfläche 2 verläuft.

Die Figur 6c zeigt einen Messarm in Form eines Lichtwellenleiters 15c und einen Referenzarm in Form eines Lichtwellenleiters 16c. Der Messarm verläuft als Lichtwellenleiter, der in eine Ausnehmung des Substrats des Messkörpers 1 mittels eines festen Materials 37 eingegossen oder eingeklebt ist. Das Material 37 ist dazu geeignet, Wärme- und/oder Druckwellen möglichst verzögerungsfrei zu leiten. Das Material 37 kann beispielsweise ein Harz und/oder ein Polymer sein. Der Lichtwellenleiter 15c kann beispielsweise ein faseroptischer Lichtwellenleiter sein. Der den Referenzarm bildende Lichtwellenleiter 16c kann ohne eine starre Kopplung an den Messkörper 1 an diesem entlang verlaufen und als faseroptischer Lichtwellenleiter ausgebildet sein.

Die Figur 6d zeigt einen Messarm in Form eines Lichtwellenleiters 15d und einen Referenzarm in Form eines Lichtwellenleiters 16d. Der Lichtwellenleiter 15d kann als integrierter Lichtwellenleiter in das Substrat des Messkörpers 1 integriert sein. Der Lichtwellenleiter 16d kann an oder in dem Messkörper 1 innerhalb einer Einbettung in ein Material 38 verlaufen, wobei das Material 38 so gestaltet ist, dass es Wärme- und oder Druckwellen schlechter leitet als das Material des Messkörpers 1 oder das Substrat des Messkörpers 1. Das Material 38 kann beispielsweise als Silikon, allgemein als Elastomer und/oder Schaumstoff ausgebildet sein.

Die Figur 6e zeigt einen Messarm in Form eines Lichtwellenleiters 15e und einen Referenzarm in Form eines Lichtwellenleiters 16e. Beide Lichtwellenleiter 15e, 16e verlaufen innerhalb des Messkörpers 1, insbesondere als in das Substrat integrierte Lichtwellenleiter, sind dabei jedoch durch eine Barriereschicht 39 getrennt. Diese besteht aus einem Material, das Wärme- und oder Druckwellen schlechter leitet als das Material des Messkörpers 1 oder das Substrat des Messkörpers. Das Material der Barriereschicht 39 kann beispielsweise als Silikon, allgemein als Elastomer und/oder Schaumstoff oder einem weichen, beispielsweise thermoplastischen Kunststoff ausgebildet sein. Die Barriereschicht 39 kann auch wenigstens abschnittsweise als Gasspalt ausgebildet sein.

Die Figur 6f zeigt einen Messarm in Form eines Lichtwellenleiters 15f und einen Referenzarm in Form eines Lichtwellenleiters 16f. Der Messarm ist zwischen einer beispielsweise schlitzförmigen Ausnehmung 40 des Messkörpers oder eines Substrats des Messkörpers 1 und der Messfläche 2 angeordnet. Der Referenzarm ist auf der der Messfläche 2 abgewandten Seite der Ausnehmung 40 angeordnet. Die Ausnehmung kann sackförmig, beispielsweise auch als Bohrung oder als eine Mehrzahl von Bohrungen ausgeführt sein. Der Messarm kann auch eine Länge aufweisen, die größer ist als die Länge des Referenzarms, dadurch, dass der Messarm wenigstens abschnittsweise in Schleifen verläuft und/oder spiralförmig oder mäanderförmig oberhalb der Ausnehmung 40 verläuft. Es kann aber zudem, wie in der Figur dargestellt, auch vorgesehen sein, dass der Referenzarm wenigstens abschnittsweise in Schleifen verläuft und/oder spiralförmig oder mäanderförmig verläuft.

Die Figur 6g zeigt einen Messarm in Form eines Lichtwellenleiters 15g und einen Referenzarm in Form eines Lichtwellenleiters 16g. Der Referenzarm ist auf der der Messfläche 2 abgewandten Seite der senkrecht zur Zeichenebene den Messkörper 1 durchsetzenden, schlitzförmigen Ausnehmung 41 angeordnet. Die Ausnehmung 41 kann auch als eine oder mehrere Bohrungen ausgeführt sein, kann jedoch auch in einer bei der Formung von Substraten üblichen Technik, beispielsweise Ätztechnik oder durch Laserschneiden oder andere abtragende Bearbeitung eingebracht sein. Die Formung eines solchen Substrats kann auch in einem additiven Verfahren vorgenommen sein (3d-Druck). Der Messarm kann eine Länge aufweisen, die größer ist als die Länge des Referenzarms, dadurch, dass der Messarm wenigstens abschnittsweise in Schleifen verläuft und/oder spiralförmig oder mäanderförmig verläuft. Es kann aber zudem, wie in der Figur dargestellt, auch vorgesehen sein, dass der Referenzarm wenigstens abschnittsweise in Schleifen verläuft und/oder spiralförmig oder mäanderförmig verläuft. Die Schleifen, Spiralen oder Mäander können jeweils in einer Ebene verlaufen, die parallel zur Messfläche 2 liegt, jedoch auch in einer Ebene, die senkrecht zur Messfläche 2 verläuft.

Die Figur 6h zeigt zwei Lichtwellenleiter 15h, 16h, zwischen denen Lichtwellen mittels des Resonanzelementes 17h in Form eines LWL- Resonanzringes gekoppelt werden können.

Die Intensität einer in den Lichtwellenleiter 15 h eingespeisten und vom Lichtwellenleiter 15h zum Lichtwellenleiter 16h oder über einen weiteren LWL- Resonanzring 19h zum LWL 18h transportierten/übergekoppelten Lichtwelle, beispielsweise gemessen am Verhältnis der Intensitäten der am LWL 16h oder 18h ausgekoppelten zur in den LWL 15h eingekoppelten Lichtwelle, hängt davon ab, wie weit die Wellenlänge der Lichtwelle von einer Resonanzwellenlänge des Resonanzelementes oder der mehreren Resonanzelemente entfernt ist. Durch eine Druck- und/oder Temperaturwelle kann das Resonanzelement/können die Resonanzelemente durch Änderung des Brechungsindex verstimmt werden, so dass das Resonanzelement/die Resonanzelemente einen effizienten Temperatur- und/oder Drucksensor darstellt/darstellen. Es können, wie in der Figur dargestellt, auch mehrere, beispielsweise wenigstens zwei, wenigstens drei oder wenigstens fünf solcher Elemente zur Erhöhung der Empfindlichkeit in Reihe geschaltet werden.

Auch eine Parallelschaltung mehrerer, beispielsweise wenigstens zwei, mehr als zwei, mehr als drei oder mehr als fünf, solcher Elemente 17i, 19i ist denkbar, wie in der Figur 6i zwischen dem Eingangs-LWL 15i und dem Ausgangs- LWL16i dargestellt. Hierdurch kann ebenfalls die Empfindlichkeit der Temperatur- und/oder Druckmessung gestaltet werden.

Bei der Verwendung von Lichtwellenleiter- Resonanzelementen kann durch eine Auswerteeinrichtung der zeitliche Verlauf der Intensität des Detektionslichts erfasst und daraus der zeitliche Verlauf der Temperatur oder des Drucks während des Durchlaufens von Druck- und /oder Wärmewellen gemessen werden. Aus dem zeitlichen Verlauf, der bei Verwendung einer Modulation periodisch sein kann, kann die Absorptionsstärke des Anregungsstrahls in dem zu analysierenden Stoff ermittelt und daraus ein Spektrum ermittelt werden. Bei demgemessenen zeitlichen Verlauf der Intensität des Detektionslichtes kann beispielsweise die Amplitude oder ein Mittelwert der Abweichung der Intensität bei eingeschaltetem, moduliertem Anregungsstrahl von der Intensität bei abgeschaltetem Anregungsstrahl ausgewertet werden.

Die Figur 6k zeigt analog zur Figur 6a einen Messarm in Form eines Lichtwellenleiters 15a und einen Referenzarm in Form eines Lichtwellenleiters 16a. Ein Strahlteiler bzw. Splitter ist mit 35 bezeichnet, während ein Koppler, in dem die Strahlen des Messarms und des Referenzarms wieder zusammengeführt sind, mit 36 bezeichnet ist. Der Referenzarm ist in einem mittleren Bereich des Messkörpers 1 von dem Messarm auf einer Länge L in einem Abstand der Größe D geführt. Der Referenzarm 16a ist auf der der Messfläche 2 abgewandten Seite des Messarms 15a angeordnet und somit von der Messfläche 2 um den Betrag D weiter entfernt als der Messarm. Mit 23 ist eine Auswerteeinrichtung bezeichnet, die die Lichtintensität hinter dem Koppler 36 erfasst und auswertet sowie dieser eine Phasenverschiebung des Detektionslichtes und damit eine Absorptionsintensität des Anregungsstrahls in dem zu analysierenden Stoff zuordnet.

Innerhalb des Messkörpers können eine oder mehrere Wärmesenken und/oder eine oder mehrere Wärmebarrieren oder keine von beiden angeordnet sein, der Messkörper 1 kann also auch homogen und frei von Wärmesenken oder Wärmebarrieren sein.

Eine Wärme- und/oder Druckwelle, die sich von dem Stoff durch die Messfläche 2 in den Messkörper 1 hinein ausbreitet, trifft zunächst auf den ersten Messabschnitt (Messarm 15a) des Interferometers und erzeugt dort eine Phasenverschiebung des Detektionslichtes. Eine Zeit t später, die sich aus der Ausbreitungsgeschwindigkeit der Welle im Messkörper und dem Abstand D ergibt, wird in dem zweiten Messarm/Referenzarm 16a des Interferometers eine Phasenverschiebung erzeugt. Bestehen beide Phasenverschiebungen eine Zeitlang gleichzeitig, so heben sich die Phasenverschiebungen auf und erzeugen keine Intensitätsänderung des Detektionslichtes. Während der Zeitintervalle, in denen die Welle jeweils nur in einem Arm/Messabschnitt 15a, 16a wirkt, eilt einmal das Detektionslicht in dem ersten Arm und darauf das Detektionslicht in dem anderen Arm voraus oder nach. Der zeitliche Verlauf dieser Entwicklung ist wegen der bekannten Ausbreitungsgeschwindigkeit der Welle im Messkörper vorhersagbar. Das Ausmaß der Intensitätsänderung des Detektionslichtes, die durch die Auswerteeinrichtung 23 nachgewiesen wird, erlaubt die Ermittlung der Amplitude der Wärme und/oder Druckwelle und damit der Absorptionsstärke des Anregungslichtes in dem zu analysierenden Stoff.

Die Figur 6l zeigt den Intensitätsverlauf I des Detektionslichtes nach Durchlaufen der Interferometrischen Einrichtung auf der vertikalen Achse, aufgetragen gegen die Zeit auf der horizontalen Achse.

Zum Zeitpunkt t1 kommt die Welle im Messkörper an dem Messarm 15 a an und verursacht dort eine Phasenverschiebung des Detektionslichts gegenüber dem Licht, das durch den Referenzarm 16a fällt. Im Ergebnis sinkt die Intensität von I1 auf I2. Zum Zeitpunkt t2 erreicht die Welle den Referenzarm 16a und verursacht dort ebenfalls eine Phasenverschiebung desselben Ausmaßes und derselben Richtung. Da der Einfluß der Welle auf den Messarm noch anhält, heben die Phasenverschiebungen sich auf, so dass keine (Teil-) Auslöschung der Lichtanteile aus den verschiedenen Armen der interferometrischen Einrichtung erfolgt. Die Intensität des Detektionslichtes erreicht nach t2 wieder den Wert I1. Darauf sinkt die Intensität nach t3, da nun nur noch in dem Referenzarm 16a eine Phasenverschiebung vorliegt und nach t4, also nachdem die -Welle durch die interferometrische Einrichtung vollständig durchgelaufen ist, stellt sich wieder die Intensität I1 ein. Aus der Differenz zwischen I1 und I2 läßt sich die Amplitude der Welle und damit die Absorptionsstärke des Anregungsstrahls in dem Stoff ermitteln.

In der Figur 6m ist eine Anordnung gezeigt, bei der der Anregungsstrahl 10 von der Anregungsstrahlquelle 4 an einer Begrenzungsfläche des Messkörpers 1 vorbei in den Stoff 3 eindringt, um dort absorbiert zu werden, was durch einen stilisierten Kreis angedeutet ist. Von dort geht die Wärme und/oder Druckwelle aus und breitet sich unter anderem in den Messkörper 1 und zu der interferometrischen Einrichtung 12 hin aus.

Zudem ist eine weitere Position der Anregungsstrahlquelle 4' eingezeichnet, von der aus der Anregungsstrahl 10' schräg an dem Messkörper 1 vorbei in den Stoff 3 eingestrahlt wird und dort unterhalb des Messkörpers 1 absorbiert wird. In diesem Fall gelangt ein noch größerer Anteil der Welle in den Messkörper 1 und zu der interferometrischen Einrichtung. Auch eine Führung des Anregungsstrahls mittels eines Lichtwellenleiters ist denkbar. An den Messkörper 1 kann ein Körper (gestrichelt eingezeichnet) aus einem anderen Material angesetzt werden, das beispielsweise für den Anregungsstrahl 10 transparent, insbesondere transparenter ist als das Material des Messkörpers 1.

In der Figur 6n ist dargestellt, dass der Messkörper 1 mit dem zu analysierenden Stoff im Bereich der Messfläche nicht nur durch einen unmittelbaren körperlichen Kontakt gekoppelt werden kann, sondern auch unter Zwischenfügung eines Mediums wie einer Zwischenschicht aus einem Festkörpermaterial oder einer Flüssigkeitsschicht oder auch einer Gasschicht.

In der Figur 6n ist konkret an der Messfläche 2 eine Ausnehmung 200 dargestellt, die optional auf der Messfläche 2 auch durch einen erhabenen Rand 201 umgeben sein kann. Es kann zur Schaffung eines Hohlraums auch nur ein umlaufender erhabener Rand auf der Messfläche vorgesehen sein. Wird die Messfläche 2 auf den zu analysierenden Stoff, beispielsweise ein Körperteil eines Lebewesens, aufgelegt, so wird zwischen dem Stoff und dem Messkörper ein Hohlraum ausgebildet, der ein akustisches Koppelelement bildet. Die Druck- und/oder Wärmewelle kann von dem Stoff in den Hohlraum eintreten und durch ein gasförmiges Medium in dem Messkörper eintreten, wo die Welle durch ein interferometrisches Element 6 nachgewiesen werden kann. Durch die hohe Empfindlichkeit der interferometrischen Messmethode kann die Welle somit auch akustisch effektiv nachgewiesen und bezüglich ihrer Intensität gemessen werden.

Der Anregungsstrahl kann von der Anregungsstrahlquelle 4 aus unmittelbar durch den Hohlraum 200 zu dem zu analysierenden Stoff geleitet werden. Dazu kann der Messkörper wenigstens teilweise eine Ausnehmung für den Anregungsstrahl aufweisen oder dieser kann durch einen Lichtwellenleiter durch den Messkörper geleitet werden. Der Anregungsstrahl kann auch wenigstens teilweise durch das Material des Messkörpers 1 geleitet werden.

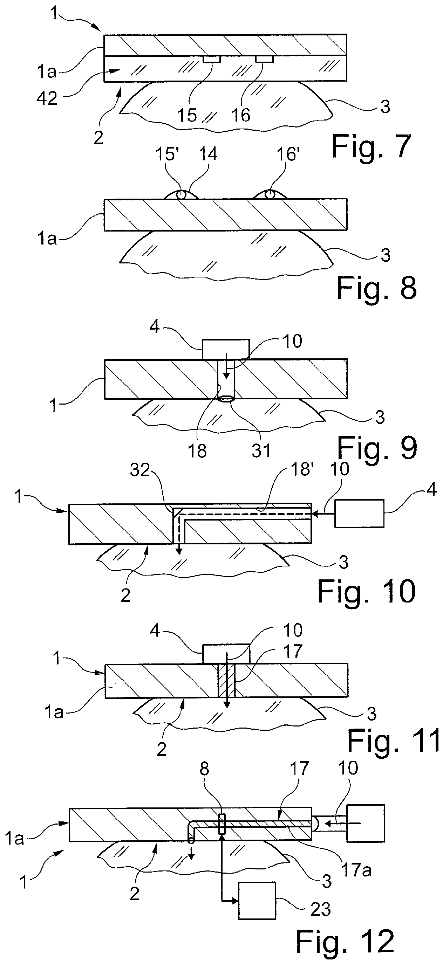

In der Figur 7 ist eine Variante des Messkörpers 1 dargestellt, in dem die Lichtwellenleiter 15, 16 der interferometrischen Anordnung auf der Seite eines Substrats 1a angeordnet sind, die der Messfläche 2 zugewandt ist. Auf dieser Seite ist das Substrat 1a mit einer Beschichtung 42 bedeckt, die die Lichtwellenleiter 15, 16 abdeckt und schützt. Durch diese Anordnung ist zumindest einer der Lichtwellenleiter 15, der den Messarm der interferometrischen Anordnung darstellt, durch eine Temperatur- und/oder Druckwelle aus dem Stoff 3 unmittelbar erreichbar. Der Referenzarm 16 sollte durch nicht dargestellte Mittel von der Wirkung der Druck- und/oder Temperaturwelle abgeschirmt werden. Der Referenzarm 16 kann beispielsweise von dem Messarm 15 ausreichend weit entfernt angeordnet sein, um von der Wirkung einer Druck- und/oder Temperaturwelle deutlich weniger beeinflusst zu werden als der Messarm.

In der Figur 8 ist eine Variante dargestellt, bei der eine interferometrische Anordnung mit faseroptischen Lichtwellenleitern 15', 16' verwirklicht ist, die mit dem Substrat 1a fest verbunden sind. In dem gezeigten Beispiel ist die Verbindung durch einen Kleber 14 realisiert. Die Lichtwellenleiter können in Nuten des Messkörpers/Substrats verlaufen.

Die Figur 9 zeigt einen Querschnitt durch einen Messkörper 1, der eine durchgehende Ausnehmung 18 in Form einer Bohrung aufweist, durch die der Anregungslichtstrahl 10 geradlinig hindurchtreten und in den zu analysierenden Stoff 3 eintreten kann. Ist der Messkörper 1 an seiner Unterseite mit einer Beschichtung 22 versehen, wie in der Figur 1 dargestellt, so kann die Ausnehmung 18 an der Beschichtung enden, sofern die Beschichtung für den Anregungsstrahl 10 transparent ist. Die Ausnehmung 18 kann jedoch auch die Beschichtung 22 durchsetzen.

In der Ausnehmung 18 kann beispielsweise ein strahlformendes Element in Form einer Linse oder eines Kollimators 31 vorgesehen sein. Die in Figur 9 gezeigte Strahlführung des Anregungsstrahls kann mit jeder Art der in den Figuren gezeigten und weiter oben erläuterten interferometrischen Einrichtung (in Figur 9 nicht dargestellt) kombiniert werden.

In der Figur 10 ist eine Anordnung dargestellt, bei der der Anregungslichtstrahl 10 durch die Lasereinrichtung 4 parallel zur Messfläche 2 in den Messkörper 1 eingestrahlt wird. Es ist eine durchgehende Ausnehmung 18' in den Messkörper 1 vorgesehen, die eine rechtwinklige Abbiegung in Richtung zur Messfläche 2 hin aufweist. Im Bereich der Richtungsänderung ist ein Reflexionselement 32 beispielsweise in Form eines Spiegels vorgesehen. Der Anregungslichtstrahl 10 kann bei der gezeigten Anordnung senkrecht durch die Messfläche 2 in dem zu analysierenden Stoff 3 eintreten. Die in Figur 10 gezeigte Strahlführung des Anregungsstrahls kann mit jeder Art der in den Figuren gezeigten und weiter oben erläuterten interferometrischen Einrichtungen (in Figur 10 nicht dargestellt) kombiniert werden.

In der Figur 11 ist ein Querschnitt durch einen Messkörper 1 gezeigt, in dem eine zweite Lichtwellenleiterstruktur 17 zur Führung des Anregungslichtstrahls 10 vorgesehen ist. Diese kann als integrierter Lichtwellenleiter ausgestaltet sein, der in ein Substrat des Messkörpers 1 integriert ist. Die zweite Lichtwellenleiterstruktur 17 ist so ausgerichtet, dass der Anregungslichtstrahl 10 senkrecht durch die Messfläche 2 hindurchgeführt wird. Es ist jedoch auch denkbar, dass der Lichtwellenleiter der zweiten Lichtwellenleiterstruktur 17 in einem Winkel von weniger als 90°, beispielsweise weniger als 70° oder weniger als 50°, auf die Messfläche 1 gerichtet ist. Die Lasereinrichtung 4 kann unmittelbar oder unter Zwischenfügung eines strahlformenden Elements, beispielsweise einer Linse (in Figur 11 nicht dargestellt) an die zweite Lichtwellenleiterstruktur 17 angekoppelt werden, es kann jedoch zur Führung des Anregungsstrahls 10 zwischen der Lasereinrichtung 4 und der zweiten Lichtwellenleiterstruktur 17 auch ein flexibler faseroptischer Lichtwellenleiter vorgesehen sein. Die in Figur 11 gezeigte Strahlführung des Anregungsstrahls kann mit jeder Art der in den Figuren gezeigten und weiter oben erläuterten interferometrischen Einrichtung (in Figur 11 nicht dargestellt) kombiniert werden.

An dem der Messfläche 2 zugewandten Ende des integrierten Lichtwellenleiters der zweiten Lichtwellenleiterstruktur 17 kann ebenfalls ein strahlformendes Element, beispielsweise eine Linse (in Figur 11 nicht dargestellt) vorgesehen sein.

In der Figur 12 ist im Rahmen der zweiten Lichtwellenleiterstruktur 17 ein aufwendiger geformter integrierter Lichtwellenleiter 17a dargestellt, der den Anregungslichtstrahl 10 führt. Der Anregungslichtstrahl 10 wird beispielsweise parallel zur Messfläche 2 in den integrierten Lichtwellenleiter 17a der zweiten Lichtwellenleiterstruktur 17 eingekoppelt und durch diesen integrierten Lichtwellenleiter 17a in eine Richtung umgelenkt, die die Messfläche 2 durchsetzt, insbesondere senkrecht durchsetzt oder auch unter einem Winkel von weniger als 90°, beispielsweise weniger als 70° oder weniger als 50°. In dem Substrat 1a ist im Bereich der zweiten Lichtwellenleiterstruktur 17 eine Modulationseinrichtung 8 integriert, die zur Intensitätsmodulation des Anregungslichtstrahls durch die Verarbeitungseinrichtung 23 dient. Die Modulationseinrichtung 8 kann beispielsweise durch ein in oder an der zweiten Lichtwellenleiterstruktur 17 angeordnetes Piezoelement oder ein Heizelement implementiert sein, das die Transparenz der zweiten Lichtwellenleiterstruktur 17 moduliert, oder durch ein MEMS-Spiegelelement zur Ablenkung des Anregungslichtstrahls 10.

Der integrierte Lichtwellenleiter 17a der zweiten Lichtwellenleiterstruktur 17, der den Anregungsstrahl 10 führt, weist Abschnitte auf, in denen er parallel zur Messfläche verläuft sowie Abschnitte, in denen er in einer Richtung auf die Messfläche 2 zu, insbesondere senkrecht zur Messfläche 2, verläuft. Eine solche Gestaltung des Lichtwellenleiters ist mit den Mitteln der integrierten Optik in einem Substrat 1a in bewährter Weise möglich.

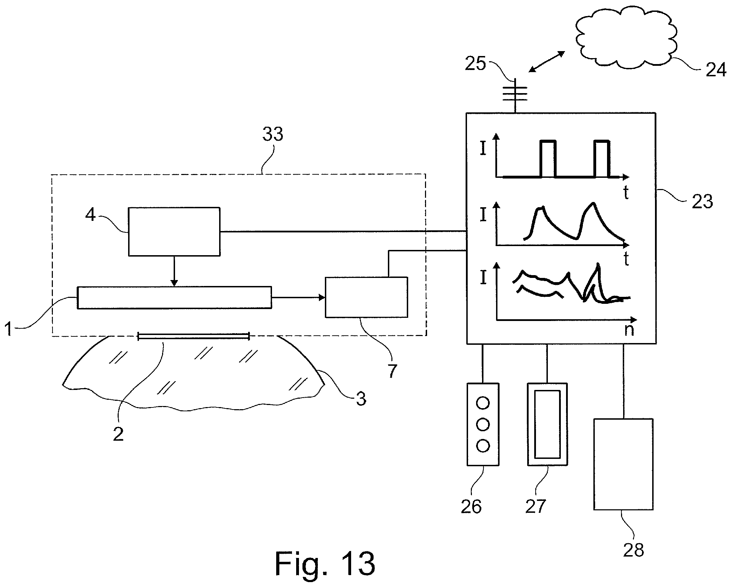

Die Figur 13 zeigt schematisch die Verarbeitung von Messdaten, die mit der Einrichtung zur Analyse eines Stoffs gewonnen werden. Im linken Teil der Figur 13 ist ein Messkörper 1 und eine Lasereinrichtung 4 zur Erzeugung eines Anregungsstrahls gezeigt, sowie eine Messeinrichtung 7. Die Messeinrichtung 7 und insbesondere auch die Lasereinrichtung 4 sind mit der Verarbeitungseinrichtung 23 verbunden, die als Microcontroller oder als Mikrocomputer ausgebildet sein kann und wenigstens einen Prozessor aufweist. In der Verarbeitungseinrichtung werden die durch die Messeinrichtung 7 erfassten Messdaten der veränderlichen Lichtintensität mit den Daten des modulierten Anregungsstrahls, d. h. mit den jeweils ausgesendeten Wellenlängen und dem Zeitverlauf der Modulation, in Beziehung gesetzt. Symbolisch sind drei Diagramme gezeigt, von denen das oberste die modulierten Laserimpulse, gegen die Zeit aufgetragen darstellt, während das mittlere Diagramm den zeitlichen Verlauf der Messdaten zeigt. Es wird jeweils beim Eintreffen einer Wärme- und/oder Druckwelle an dem interferometrischen Element, beispielsweise durch Ein/Ausschalten oder Modulation des Anregungsstrahls, dieses durch Änderung des Brechungsindex verstimmt, oder es wird eine Auslöschung oder teilweise Auslöschung der Wellenanteile aus verschiedenen Messarmen eines Interferometers bewirkt. Dadurch ändert sich die Intensität des Detektionslichts nach Durchlaufen des interferometrischen Elementes. In diesem zeitlichen Verlauf wird außer der Absorptionsstärke des Anregungsstrahls in dem zu analysierenden Stoff auch die Mischeigenschaft der Signale widergespiegelt, die für jede Periode der Modulation des Anregungsstrahls als Mischung von Signalen aus unterschiedlichen Tiefen unter der Stoffoberfläche zu der Einrichtung ausgesandt werden und die durch die Laufzeitunterschiede ein bestimmtes Abklingverhalten der Messsignale jeweils nach einem Laserimpuls erzeugen. Die Signale aus unterschiedlichen Tiefen müssen nicht voneinander getrennt werden, jedoch kann dies durch verschiedene Auswertemethoden geschehen, die an anderer Stelle in diesem Text erläutert werden.

Das dritte und unterste Diagramm zeigt eine Spektraldarstellung, in der gemessene Lichtintensitäten über den eingestrahlten Wellenlängen oder Wellenzahlen des Anregungslichtstrahls in jeweils mehreren Spektren aufgetragen sind.

Aus diesen Daten lassen sich beispielsweise physiologische Werte eines Patienten gewinnen, die aus der Konzentrationsmessung bestimmter Substanzen im Körpergewebe oder in einer Körperflüssigkeit gewonnen werden. Ein Beispiel hierfür ist die Blutzuckermessung, bei der die Glukosekonzentration in einem Körperteil gemessen wird. Gemäß der Figur 13 können Messwerte oder vorverarbeitete Werte mittels einer Kommunikationseinrichtung 25 mit einem entfernten Computer oder einem verteilten Computersystem (Cloud) abgeglichen werden. Beispielsweise können aus der Cloud oder einem entfernten Computer Referenzwerte zur Interpretation der Messwerte importiert werden. Die Referenzwerte können von der Identität des Patienten und für ihn individuell gespeicherten und abrufbaren Daten abhängen. Hierzu ist entweder die Identität des Patienten in die Verarbeitungseinrichtung 23 einzugeben oder sie ist durch gesonderte Messungen, beispielsweise durch einen Fingerabdrucksensor, der in die Messeinrichtung integriert sein kann, festzustellen.

Innerhalb der Cloud kann auch ein Vergleich mit Messwerten anderer Patienten oder ein Vergleich mit früheren Messwerten desselben Patienten durchgeführt werden, wobei auch Umgebungsbedingungen wie die Temperatur, der Luftdruck oder die Luftfeuchtigkeit am Aufenthaltsort des Patienten mitberücksichtigt werden können. Sensoren zur Erfassung dieser Werte können in die Vorrichtung/Einrichtung zur Analyse eines Stoffs gemäß der Erfindung integriert sein.

Als Verarbeitungsergebnis kann die Verarbeitungseinrichtung 23 durch eine erste Ausgabeeinrichtung 26 eine tendenzielle Information, beispielsweise drei- oder fünfstufig, in Form von Angaben wie optimal, gut, vertretbar, noch gut, besorgniserregend oder in Form von Farben oder Symbolen ausgeben. In einer weiteren Ausgabeeinrichtung 27, die eine konkrete Werteanzeige ermöglicht, können Messwerte an einem Bildschirm oder in einer Digitalanzeige ausgegeben werden. Zudem können Messwerte oder Messwerttendenzen an ein Programmmodul 28 ausgegeben werden, das beispielsweise auch in einem gesonderten mobilen Verarbeitungsgerät wie einem Mobiltelefon ablaufen kann. In diesem Gerät können die ausgewerteten Messergebnisse dann beispielsweise zur Zusammenstellung einer einzunehmenden Mahlzeit oder zur Auswahl von zur Verfügung stehenden Nahrungsmitteln sowie einer Nahrungsmittelmenge verwendet werden. Es kann auch eine Empfehlung zum Verzehr bestimmter Lebensmittel mit einer Mengenangabe erfolgen. Dies kann beispielsweise mit einem Zubereitungsvorschlag verbunden sein, der aus einer Datenbank abgerufen und insbesondere auch in elektronische Form übermittelt werden kann. Diese Zubereitungsanweisung kann auch an eine automatische Vorrichtung zur Nahrungszubereitung übermittelt werden.

Es kann durch die Ausgabeeinrichtung/Anzeige 27 oder eine zu dieser parallelen Signaleinrichtung in einer Ausführungsform ein Vorschlag für eine Insulindosis abhängig von weiteren Patientenparametern (z.B. Insulinkorrekturfaktor) oder eine automatische Signalübertragung an eine Dosiereinrichtung in Form einer Insulinpumpe erfolgen.

Die Verarbeitungseinrichtung 23 kann in das Gehäuse 33 der Einrichtung mit integriert werden, sie kann jedoch auch gesondert vorgesehen werden, beispielsweise in einem mobilen Computer oder einem Mobilfunkgerät. Es ist für diesen Fall eine Kommunikationsschnittstelle zwischen den in dem Gehäuse 33 angeordneten Komponenten, insbesondere der Messeinrichtung 7 und der Verarbeitungseinrichtung 23, beispielsweise über einen Funkstandard, vorzusehen. Das Gehäuse 33 kann als tragbares Gehäuse, beispielsweise auch als am Handgelenk eines Menschen nach Art einer Armbanduhr (wearable) tragbares Gehäuse ausgebildet sein. In einer weiteren Ausführungsform kann auch die Lasereinrichtung außerhalb des Gehäuses angeordnet und für eine Messung ankoppelbar sein. Die Ankopplung kann beispielsweise mittels eines faseroptischen Lichtwellenleiters realisiert sein und/oder dadurch, dass der Anregungsstrahl der Lasereinrichtung durch Anlegen der Lasereinrichtung an eine Referenzfläche des Gehäuses relativ zum Messkörper für eine Messung geeignet ausgerichtet wird.

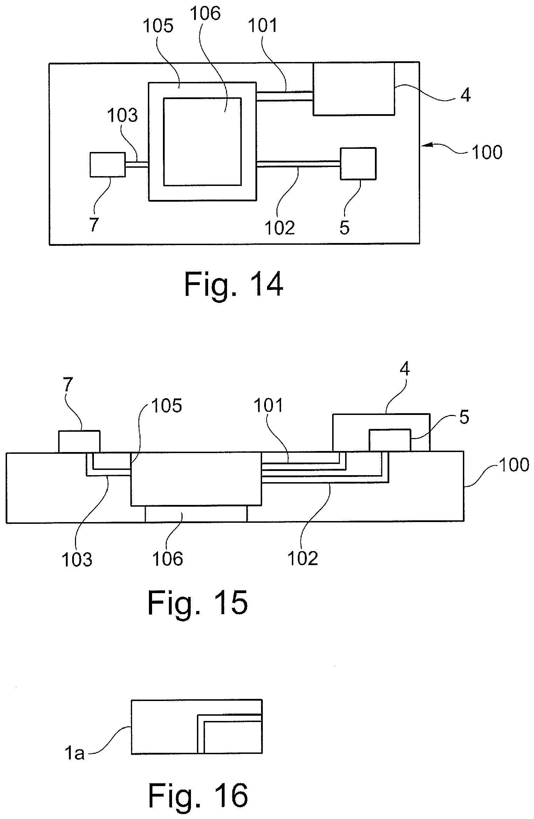

Die Figur 14 zeigt in einer Draufsicht ein Substrat 100, das eine Anregungslichtquelle 4, beispielsweie in Form einer Lasereinrichtung, insbesondere eines Laserarrays trägt. Zudem trägt das Substrat 100 eine Detektionslichtquelle 5 und eine Messeinrichtung 7, beispielsweise in Form eines strahlungsempfindlichen Halbleiterbauelementes für die Messung der Inensität des Detektionslichts. Jedes der Elemente 4, 5, oder 7 kann auch ganz oder teilweise in eine Halbleiterstruktur des Substrats 100 integriert oder aus diesem durch mikromechanische Fertigungstechnik und beispielsweise Dotierung herausgearbeitet sein. Das Substrat weist Lichtwellenleiter 101, 102, 103 auf, die entweder ganz oder teilweise in das Substrat integriert oder als faseroptische LWL an diesem befestigt sind, beispielsweise in V-Nuten, die die Lichtwellenleiter ausreichend genau positionieren. Der Lichtwellenleiter 101 führt das Anregungslicht/die Anregungsstrahlung, während die Lichtwellenleiter 102, 103 das Detektionslicht/die Detektionsstrahlung führen.

Das Substrat 100 weist, wie besonders in Figur 15 ersichtlich ist, eine mikromechanisch genau eingepasste Ausnehmung 105 auf, in die ein weiteres Substrat 1a derart eingepasst werden kann, dass einer oder mehrere der Lichtwellenleiter 101, 102, 103 direkt vor entsprechenden Anschlusslichtwellenleitern (nicht gezeigt) des weiteren Substrats enden, so dass die geführte Strahlung unmittelbar in die Lichtwellenleiter des weiteren Substrats 1a eingekoppelt und aus diesen zu dem Lichtwellenleiter der Messeinrichtung/dem Detektor 7 wieder ausgekoppelt werden kann. Zu diesem Zweck können jeweils auch Koppelelemente vorgesehen sein, die die Effizienz der Kopplung erhöhen. Das Substrat 1a weist dann beispielsweise, wie in Figur 16 und in der vergleichbaren Figur 12 gezeigt, einen integrierten Lichtwellenleiter auf, der das Anregungslicht zur Messfläche führt. Zudem weist das Substrat 1a ein integriertes interferometrisches Element mit integrierten Anschlusslichtwellenleitern auf. Die Messfläche kann auf einer der beiden Seiten der Substrate 100, 1a liegen. Liegt die Messfläche auf der in der Figur 15 unteren Seite, so kann in dem Substrat 100 innerhalb der Ausnehmung 105 ein Fenster 106 als durchgehende Ausnehmung vorgesehen sein.

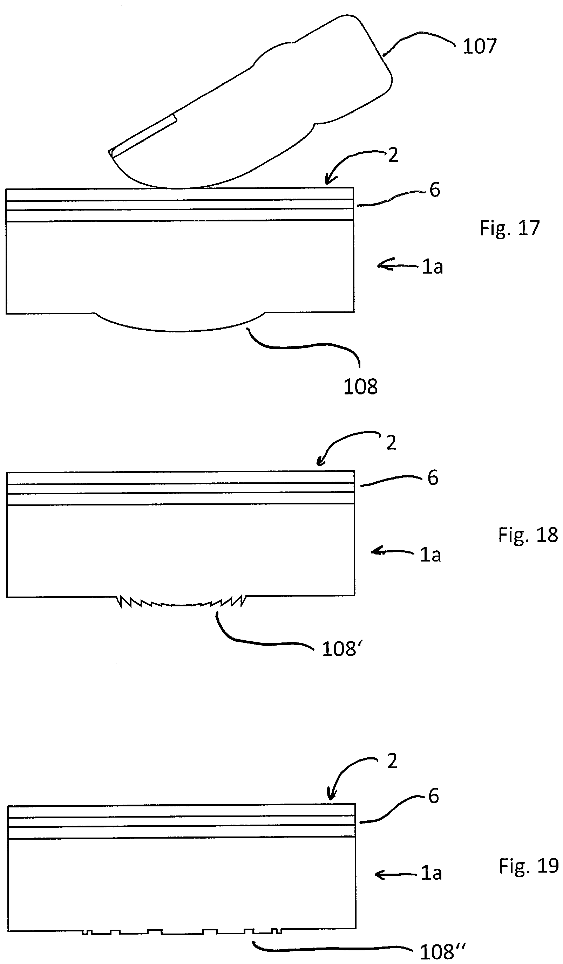

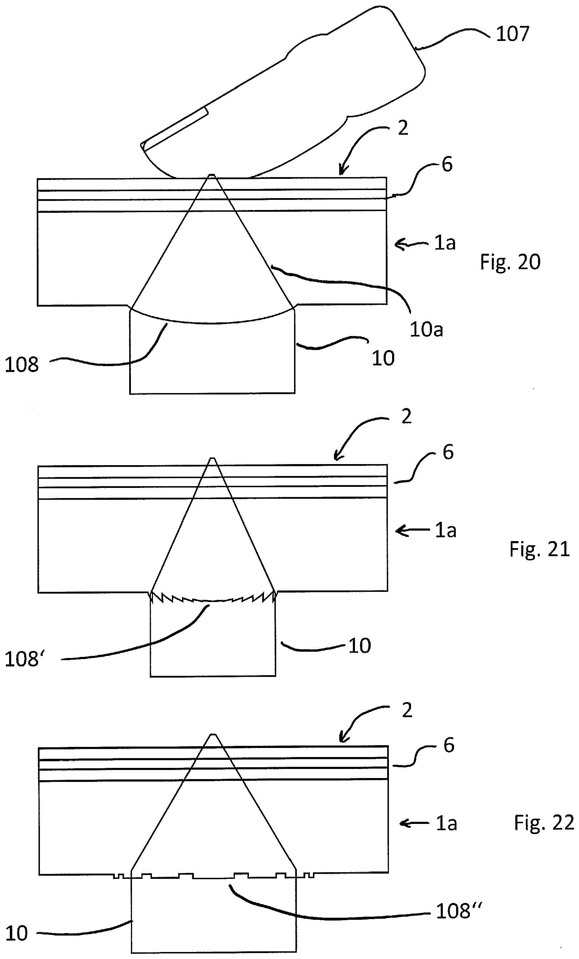

Die Figur 17 zeigt, ebenso wie die Figuren 18 bis 22, in einer Querschnittsdarstellung ein Substrat 1a, in das eine erste Lichtwellenleiteranordnung 6 als Teil einer Detektionseinrichtung eingebettet ist. Schraffuren von geschnittenen Partien sind der Übersichtlichkeit weggelassen. Die Messfläche 2 liegt jeweils in dem in den Figuren oberen Teil des Substrats 1a. Zur Anschauung ist in der Figur 17 ebenso wie in der Figur 20 ein menschlicher Finger 107 als beispielhaftes Messobjekt dargestellt, dessen Substanz analysiert werden soll. Der Finger wird jeweils zur Analyse auf die Messfläche 2 aufgelegt.

In den Figuren 17 bis 22 sind jeweils Substrate gezeigt, deren Material für einen Anregungsstrahl 10 im Infrarotbereich oder allgemein in dem Wellenlängenbereich des Anregungsstrahls durchlässig oder zumindest teilweise durchlässig ist. Dies gilt beispielsweise für ein Substrat aus Silizium für den Infrarotbereich. Ein Anregungsstrahl kann deshalb durch das Substratmaterial oder zumindest durch begrenzte Schichtdicken hindurch auf die Messfläche und durch diese hindurch in den zu vermessenden Stoff gerichtet werden. Es ist in einem solchen Fall nicht notwendig, für den Anregungsstrahl 10 eine durchgehende Öffnung im Substrat vorzusehen. Der Anregungsstrahl kann an der ersten Lichtwellenleiterstruktur 6 vorbei oder durch diese hindurch gelenkt werden. Es kann auch ein Teil der Strecke, die der Anregungsstrahl im Messkörper zurücklegt, innerhalb einer Ausnehmung/eines Hohlraums liegen. Hierzu kann eine Ausnehmung zumindest abschnittsweise in dem Messkörper vorgesehen werden. Im Bereich der Messfläche kann dann beispielsweise eine dünne Schicht des Substrats stehen bleiben. Es kann aber auch abschnittsweise in dem Messkörper ein Materialeinsatz in Form eines Lichtwellenleiters aus einem Material vorgesehen sein, das für den Anregungsstrahl durchlässiger ist als das Material des Substrats.

In den Figuren 17-22 und 23-25 werden verschiedene Gestaltungen der Führung des Anregungsstrahls dargestellt. Die Detektionseinrichtung ist der Übersichtlichkeit halber jeweils weggelassen. Selbstverständlich sind alle beschriebenen Gestaltungen der Detektionseinrichtung in Kombination mit den in den Figuren 17- 25 gezeigten Gestaltungen der Führung des Anregungsstrahls realisierbar.

An der der Messfläche 2 gegenüberliegenden Seite des Messkörpers oder des Substrats 1a ist in das Substrat jeweils eine Linse 108, 108', 108" integriert, insbesondere durch das Material des Substrats geformt und beispielsweise durch abtragende Verfahren, insbesondere durch Ätzen aus dem Material des Substrats herausgearbeitet.

Es sind in den Figuren 17 bis 22 drei Beispiele für mögliche Linsenformen dargestellt, wobei die erste Linse in den Figuren 17 und 20 dargestellt ist, die zweite Linse in den Figuren 18 und 21 und die dritte Linse in den Figuren 19 und 22.

Die erste Linse 108 entspricht einer normal brechenden, refraktiven konvexen Sammellinse, die zweite Linse 108' entspricht einer Sammellinse (refraktiv) im Fresnellschliff (Kinoformlinse) und die dritte Linse entspricht einer diffraktiven Linse, die eine Bündelung des Anregungsstrahls 10 durch Beugung an einer konzentrischen Gitterstruktur bewirkt.

Die optischen Achsen der Linsen können jeweils senkrecht auf der Messfläche 2 stehen, so dass eine Anregungslichtquelle unmittelbar gerade durch das Substrat 1a hindurchstrahlen kann. Jedoch können die optischen Achsen auch gegenüber der Senkrechten zur Messfläche 2 schräggestellt sein, um eine möglicherweise platzsparende Positionierung der Anregungslichtquelle schräg zum Substrat zu erlauben.

In den Figuren 20, 21, 22 sind jeweils die Linsenformen 108, 108', 108" am Substrat 1a mit den Anregungssstrahlen 10 und den auf den zu analysierenden Stoff fokussierten Strahlenbündeln 10a dargestellt.

In den Figuren 17-22 sind jeweils interferometrische Elemente in der Nähe der Messfläche im Substrat vorgesehen, jedoch soll in diesen Figuren hauptsächlich die Strahlführung des Anregungsstrahls 10 gezeigt werden.

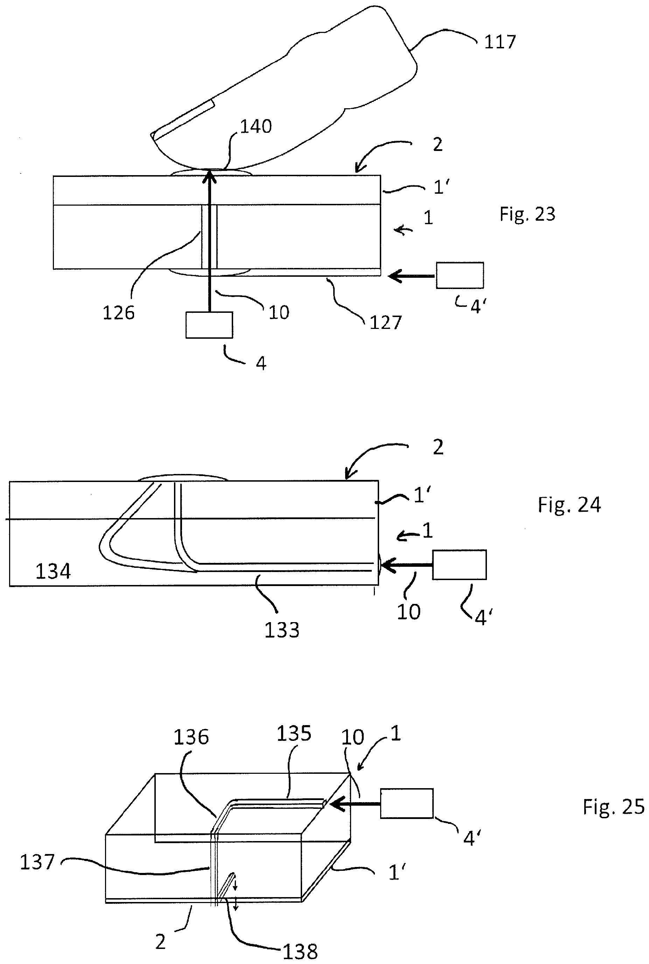

In der Figur 23 ist ein Messkörper 1 mit einer Sensorschicht 1' im Querschnitt dargestellt, bei dem ein Anregungsstrahl 10 von der Laseranordnung 3 aus in einen Lichtwellenleiter 126 geleitet wird, der den Messkörper 1 bis zur Schicht 1' durchsetzt. Der Lichtwellenleiter 126 kann auch durch die Schicht 1' hindurch bis zur Messfläche 2 reichen, jedoch kann auch vorgesehen sein, dass entweder die Schicht 1' eine Aussparung für den Anregungsstrahl 10 aufweist oder der Anregungsstrahl durch das Material der Schicht 1' hindurchtritt. Auch von dem Substrat, welches im gezeigten Ausführungsbeispiel den Messkörper 1 bildet, kann eine gewisse Schichtdicke vor der Messfläche oder vor der Schicht 1' stehen bleiben und vom Anregungsstrahl 10 durchquert werden. Im Bereich der Messfläche 2, beispielsweise unmittelbar an die Messfläche 2 angrenzend und/oder innerhalb der Schicht 1', kann eine Linse 140 zur Fokussierung des Anregungsstrahles 10 auf einen Punkt in dem zu untersuchenden Stoff vorgesehen sein. Der Lichtwellenleiter 126 verläuft gerade von der Laservorrichtung 4 bis zur Messfläche 2 und durchsetzt die Detektionseinrichtung in Form eines nicht näher dargestellten interferometrischen Elements in der Schicht 1' oder im Substrat 1. Ein Lichtwellenleiter kann auch, beispielsweise wenn die Lasereinrichtung 4' seitlich neben dem Messkörper angeordnet ist, teilweise oder vollständig an und entlang der Oberfläche des Messkörpers 1 verlaufen (vgl. Figur 25). In der Figur 23 verläuft der Lichtwellenleiter 127 von der Lasereinrichtung 4' zunächst auf einem ersten Teil seiner Länge an oder auf der Oberfläche des Messkörpers, um dann weiter auf einem zweiten Teil seiner Länge wie der Lichtwellenleiter 126 durch den Messkörper hindurch zu verlaufen. Der Anregungsstrahl kann im Bereich der Richtungsänderung des Lichtwellenleiters beispielsweise an einem Spiegel reflektiert werden oder der Lichtwellenleiter kann dort gekrümmt sein. Ein solcher Lichtwellenleiter 126, 127 kann in das Material des Messkörpers 1 fertigungstechnisch integriert (beispielsweise durch SOI- Silizium on Insulator Technologie) oder als faseroptischer Lichtwellenleiter mit diesem beispielsweise durch Verkleben verbunden sein, oder der Lichtwellenleiter kann auf einem Teil seiner Länge integriert und auf einem anderen Teil seiner Länge als faseroptischer Lichtwellenleiter ausgeführt sein.

Es kann, wie aus der Figur 24 in zwei verschiedenen Varianten der Lichtwellenleitergestaltung ersichtlich ist, auch ein gekrümmter Lichtwellenleiter 133, 134, vorgesehen sein, der den Anregungsstrahl 10 von einer Position an dem Messkörper 1, an der die Lasereinrichtung vorgesehen ist, zur Messfläche 2 führt. Dadurch, dass die Führung des Lichtwellenleiters 133, 134 relativ frei gestaltet werden kann, kann zwischen dem von dem Anregungsstrahl durchsetzten Bereich und dem Detektionsbereich ein Mindest-Abstand gehalten werden. Der Anregungsstrahl 10 kann auch schräg zur Messfläche 2, beispielsweise in einem Winkel zwischen 0 Grad und 60 Grad, insbesondere zwischen 0 und 45 Grad zur Flächennormalen der Messfläche 2 auf die Messfläche 2 treffen und durch diese hindurchtreten.

Wegen der geringen Eindringtiefe in den zu analysierenden Stoff liegt der Bereich des Stoffes, in dem der Anregungsstrahl 10 mit diesem wechselwirkt, trotz einer schrägen Einstrahlrichtung unmittelbar unterhalb der Detektionseinrichtung, die beispielweise in Form eines interferometrischen Elementes vorliegen kann. Die gekrümmten Lichtwellenleiter 133, 134 können beispielsweise wenigstens abschnittsweise als faseroptische Lichtwellenleiter in einer Bohrung oder ähnlichen Aussparung des Messkörpers 1 verlegt und dort eingeklebt oder eingegossen sein.

Es kann auch, wie aus der Figur 25 gezeigt, zur Führung des Anregungsstrahls 10 ein Lichtwellenleiter 135, 136, 137, 138 vorgesehen sein, der beispielsweise in mehreren Richtungen und/oder in zwei oder drei aufeinander senkrecht stehenden Richtungen an einer oder zwei oder drei verschiedenen, aneinander angrenzenden Oberflächen des Messkörpers 1 entlang geführt ist. Ein solcher Lichtwellenleiter 135, 136, 137, 138 kann beispielsweise, wie auch die in den Figuren 23 und 24 gezeigten Lichtwellenleiter, in die jeweiligen Messkörper integriert sein. An den Oberflächen eines Messkörpers ist dies in SOI- Technologie oder je nach dem Material des Messkörpers 1 in einer verwandten Festkörper-Herstellungstechnologie besonders einfach möglich. In einem Silizium-Substrat kann dazu ein Lichtwellenleiter eingebracht sein, der durch Siliziumoxidschichten oder andere Schichten abgedeckt und von dem Substrat getrennt ist. Dazu kann zunächst in das Substrat eine passende Ausnehmung geätzt oder gesputtert werden, um danach das Material der Abdeckung und des Lichtwellenleiters geeignet abzuscheiden. Die Abdeckung des Lichtwellenleiters kann in diesem Fall beispielsweise mit der Oberfläche des Messkörpers bündig abschließen, so dass der Lichtwellenleiter nicht über den Messkörper 1 hinausragt. Durch den Verlauf des Lichtwellenleiters 135, 136, 137, 138 entlang den Oberflächen des Messkörpers wird eine Wechselwirkung des Anregungsstrahls mit der Detektionseinrichtung vermieden. Der letzte Lichtwellenleiter 138 endet dann in dem Bereich, in dem der Anregungsstrahl 10 in den zu analysierenden Stoff 3 eintreten soll. Dort kann am Ende des Lichtwellenleiters 138 ein Element vorgesehen sein, das den Anregungsstrahl in den Stoff 3 hinein lenkt, beispielsweise ein Spiegel.

In dem unten rechts in der Figur 25 in einem Kreis 142 dargestellten Ausschnitt der Figur 25 ist dargestellt, dass der Lichtwelleneiter 138 auch in einer schräg auf die Messfläche 2 zulaufenden gestrichelt dargestellten Nut des Messkörpers 1 angeordnet werden kann, so dass die Längsachse des Lichtwellenleiters parallel zu dem Boden 141 der Nut durch die Messfläche 2 hindurch auf den zu analysierenden Stoff 3 ausgerichtet ist.

Die Erfindung ist in den anhängenden Ansprüchen definiert.

## Patentansprüche

1. Einrichtung zur Analyse eines Stoffes mit:
- einem Messkörper (1, 1a), der eine Messfläche (2) aufweist und im Bereich der Messfläche zur Messung wenigstens teilweise mit dem Stoff (3) zu koppeln, insbesondere unmittelbar oder mittels eines Mediums, insbesondere eines Fluids zu koppeln oder unmittelbar oder auch mittels eines Mediums in Kontakt zu bringen ist, wobei die Messfläche (2) als eine ebene Fläche gestaltet ist oder eine konkave Fläche oder Teilfläche aufweist,
- einer Anregungsstrahlquelle, die in der Lage ist, einen Anregungsstrahl verschiedener Wellenlängen zu erzeugen, insbesondere einer Lasereinrichtung (4), insbesondere mit einem Quantenkaskadenlaser QCL, einem durchstimmbaren QCL, und/oder mit einem Laserarray, vorzugsweise einem Array von QCLs, zur Erzeugung eines oder mehrerer Anregungsstrahlen (10) mit verschiedenen Wellenlängen, vorzugsweise im infraroten oder mittleren infraroten Spektralbereich, wobei der Anregungsstrahl auf den Stoff (3) gerichtet ist, wenn der Messkörper (1, 1a) im Bereich der Messfläche (2) mit dem Stoff (3) gekoppelt und/oder in Kontakt ist,
- einer Modulationseinrichtung (8) zur Intensitätsmodulierung des Anregungsstrahls (10) mit einer Modulationsrate zwischen 1 Hz und 10 kHz, und
- einer wenigstens teilweise in den Messkörper (1, 1a) integrierten oder mit diesem verbundenen Detektionsvorrichtung (5, 6, 7), die folgendes umfasst:
• eine Quelle (5) für Detektionslicht, vorzugsweise kohärentes Detektionslicht (11) sowie
• eine mit der Quelle für das Detektionslicht verbindbare oder verbundene und das Detektionslicht führende erste Lichtwellenleiterstruktur (6), deren Brechungsindex wenigstens abschnittsweise von der Temperatur und/oder dem Druck abhängig ist, wobei die erste Lichtwellenleiterstruktur wenigstens einen Abschnitt (9) aufweist, in dem die Lichtintensität von einer Phasenverschiebung von Detektionslicht in wenigstens einem Teil der ersten Lichtwellenleiterstruktur (6) durch eine Temperatur- oder Druckänderung infolge von Wärme- und/oder Temperaturwellen abhängt, die durch Absorption des Anregungsstrahls in dem Stoff (3) hervorgerufen werden, wenn der Messkörper (1, 1a) im Bereich der Messfläche (2) mit dem Stoff (3) gekoppelt ist, und
• eine Messeinrichtung (7) zur direkten oder indirekten Erfassung der Lichtintensität in dem genannten Abschnitt (9) der ersten Lichtwellenleiterstruktur (6), in dem die Lichtintensität von der genannten Phasenverschiebung von Detektionslicht im genannten wenigstens einen Teil der ersten Lichtwellenleiterstruktur durch eine Temperatur- oder Druckänderung abhängt,
wobei der Messkörper (1,1a) ein Substrat darstellt oder enthält und die erste Lichtwellenleiterstruktur (6) mindestens einen Lichtwellenleiter (15, 16) umfasst, der in das Substrat (1a) integriert ist.

2. Einrichtung nach Anspruch 1, **dadurch gekennzeichnet, dass** zumindest ein Abschnitt einer Projektion der ersten Lichtwellenleiterstruktur (6) in Richtung der Flächennormalen der Messfläche (2) mit dieser Messfläche (2) überlagert ist.

3. Einrichtung nach Anspruch 1 oder 2, mit einem Gehäuse (33), welches als tragbares Gehäuse, insbesondere als am Handgelenk eines Menschen nach Art einer Armbanduhr tragbares Gehäuse ausgebildet ist, wobei die Anregungsstrahlquelle (4) vorzugsweise eine Lasereinrichtung ist, die außerhalb des Gehäuses (33) angeordnet und für eine Messung ankoppelbar ist, wobei die Ankopplung insbesondere mittels eines faseroptischen Lichtwellenleiters realisiert ist.

4. Einrichtung nach Anspruch 1, 2, oder 3, **dadurch gekennzeichnet, dass** die Detektionseinrichtung eine interferometrische Einrichtung, insbesondere ein Interferometer (12) und/oder ein Lichtwellenleiter-Resonanzelement, insbesondere einen Resonanzring (13) oder eine Resonanzplatte aufweist.

5. Einrichtung nach einem der Ansprüche 1 bis 4, **dadurch gekennzeichnet, dass** die erste Lichtwellenleiterstruktur (6), insbesondere eine interferometrische Einrichtung der ersten Lichtwellenleiterstruktur, wenigstens einen Glasfaser-Lichtwellenleiter (14) aufweist, der wenigstens abschnittsweise mit dem Messkörper (1) fest verbunden ist.

6. Einrichtung nach einem der Ansprüche 1 bis 5, **dadurch gekennzeichnet, dass** die erste Lichtwellenleiterstruktur (6) insbesondere wenigstens einen Silizium-Lichtwellenleiter aufweist, der mit einem isolierenden Substrat verbunden oder in ein isolierendes Substrat integriert ist, und wobei weiter insbesondere der Silizium-Lichtwellenleiter wenigstens teilweise durch einen Isolator, insbesondere SiO2 abgedeckt ist.

7. Einrichtung nach einem der vorhergehenden Ansprüche, **dadurch gekennzeichnet, dass** der Anregungsstrahl (10) das Material des Messkörpers (1, 1a) , insbesondere im Bereich der Messfläche des Messkörpers oder einen der Messfläche (2) benachbarten Bereich oder einen an die Messfläche unmittelbar angrenzenden Bereich durchsetzt, wobei der Messkörper oder der von dem Anregungsstrahl (10) durchsetzte Bereich für den Anregungsstrahl transparent ist.

8. Einrichtung nach einem der vorhergehenden Ansprüche, **dadurch gekennzeichnet, dass** der Anregungsstrahl (10) innerhalb des Messkörpers (1, 1a) oder am Messkörper durch eine zweite Lichtwellenleiterstruktur (17) geführt ist.

9. Einrichtung nach einem der vorhergehenden Ansprüche, **dadurch gekennzeichnet, dass** der Anregungsstrahl (10) zwischen der Lasereinrichtung (4) und dem zu analysierenden Stoff (3) eine durchgehende Ausnehmung (18) des Messkörpers (1, 1a) durchsetzt, wobei die Ausnehmung insbesondere in einem Abstand vor der Messfläche endet oder die Messfläche (2) durchsetzt oder in einem Bereich angeordnet ist, der der Messfläche unmittelbar benachbart ist und/oder an diese angrenzt.

10. Einrichtung nach einem der vorhergehenden Ansprüche, **dadurch gekennzeichnet, dass** der Messkörper (1, 1a) als Flachkörper, insbesondere als planparalleler Körper in Form einer Platte ausgebildet ist, wobei insbesondere die Dicke des Messkörpers in Richtung senkrecht zur Messfläche (2) weniger als 50% der geringsten Ausdehnung des Messkörpers in einer in der Messfläche verlaufenden Richtung beträgt, insbesondere weniger als 25%, weiter insbesondere weniger als 10%.

11. Einrichtung nach einem der vorhergehenden Ansprüche, **dadurch gekennzeichnet, dass** der Messkörper (1, 1a) eine Spiegeleinrichtung (19) zur Reflexion des von der Lasereinrichtung (4) eingestrahlten Anregungsstrahls (10) auf die Messfläche (2) aufweist oder trägt.

12. Einrichtung nach einem der vorhergehenden Ansprüche, **dadurch gekennzeichnet, dass** der Anregungsstrahl (10) parallel zur Messfläche (2) oder in einem Winkel von weniger als 30 Grad, insbesondere weniger als 20 Grad, weiter insbesondere weniger als 10 Grad oder weniger als 5 Grad zur Messfläche in den Messkörper (1, 1a) ausgerichtet ist und dass der Anregungsstrahl in Richtung der Messfläche umgelenkt oder abgelenkt wird, wobei der Anregungsstrahl insbesondere die Messfläche oder eine gedachte Fortsetzung der Messfläche im Bereich einer durchgehenden Ausnehmung (18) in dem Messkörper durchsetzt.

13. Einrichtung nach einem der vorhergehenden Ansprüche, **dadurch gekennzeichnet, dass** in dem Messkörper (1, 1a) von der Messfläche (2) aus gesehen hinter und/oder neben der Detektionsvorrichtung (5, 6, 7), insbesondere der ersten Lichtwellenleiterstruktur (6), insbesondere an diese angrenzend und mit dieser im thermischen Kontakt stehend wenigstens eine Wärmesenke (20) in Form eines festen Körpers oder Materials angeordnet ist, wobei insbesondere die spezifische Wärmekapazität und/oder spezifische Wärmeleitfähigkeit des Körpers oder des Materials der Wärmesenke größer ist als die spezifische Wärmekapazität und/oder spezifische Wärmeleitfähigkeit des Materials der Detektionseinrichtung (5, 6, 7) und/oder der ersten Lichtwellenleiterstruktur und/oder des Substrats (1a) der ersten Lichtwellenleiterstruktur (6) und/oder der übrigen Materialien, aus denen der Messkörper (1, 1a) besteht und/oder dass in dem Messkörper (1, 1a) eine Barriere (30, 40, 41) vorgesehen ist, die den Einfluß der Wärme- und/oder Druckwelle von einem Teil der Detektionseinrichtung, insbesondere einem Teil der ersten Lichtwellenleiterstruktur (6), weiter insbesondere einem Referenzarm eines Interferometers, wenigstens teilweise fernhält, und/oder dass die erste Lichtwellenleiterstruktur (6) der Detektionseinrichtung wenigstens zwei Mess-Abschnitte (15a, 16a) aufweist, die insbesondere an verschiedenen Armen eines Interferometers angeordnet sind und in denen der Brechungsindex sich in Abhängigkeit von Druck- und/oder Temperaturänderungen, insbesondere von einer Druck-und/oder Wärmewelle ändert, so dass eine Phasenverschiebung des die Mess-Abschnitte durchlaufenden Detektionslichts und in deren Folge eine Intensitätsänderung des Detektionslichts in einem weiteren Abschnitt in Abhängigkeit von Druck- und/oder Temperaturänderungen erfolgt, wobei die beiden Messabschnitte derart in dem Messkörper angeordnet sind, dass sie durch eine Druck-und/oder Wärmewelle, die sich ausgehend von der Messfläche (2), insbesondere von dem Bereich der Messfläche, in dem der Anregungsstrahl diese durchsetzt, durch den Messkörper ausbreitet, zeitlich nacheinander, insbesondere in zeitlich gegeneinander verschobenen Zeitabschnitten oder mit einem zeitlichen Abstand passiert werden.

14. Verfahren zum Betrieb einer Einrichtung nach einem der vorhergehenden Ansprüche, **dadurch gekennzeichnet, dass** ein modulierter Anregungsstrahl (10), insbesondere durch den Messkörper hindurch, auf den zu analysierenden Stoff (3) gerichtet wird und dass durch die Detektionseinrichtung ein Lichtintensitätsverlauf oder eine periodische Lichtintensitätsänderung erfasst wird, wobei diese für eine Mehrzahl von Wellenlängen des Anregungsstrahls durch die Messung der Lichtintensitätsänderung in der ersten Lichtwellenleiterstruktur oder durch die Messung der Lichtintensität von aus der ersten Lichtwellenleiterstruktur ausgekoppeltem Licht erfasst werden und aus den erfassten Daten ein Absorptionsspektrum des zu analysierenden Stoffs gewonnen wird.

15. Verfahren nach Anspruch 14, **dadurch gekennzeichnet, dass** die Messung für verschiedene Modulationsfrequenzen des Anregungsstrahls (10) durchgeführt wird und dass aus der Verknüpfung von gewonnenen Absorptionsspektren ein korrigiertes Absorptionsspektrum ermittelt wird.

## Claims

1. Device for analysing a substance, having:
- a measuring body (1, 1a), which has a measuring surface (2) and is to be at least partially coupled with the substance (3) in the area of the measuring surface for measurement, in particular directly or by means of a medium, in particular a fluid, or is to be brought into contact with it directly or else by means of a medium, wherein the measuring surface (2) is formed as a plane surface or comprises a concave surface or partial surface,
- a source of excitation radiation capable of generating an excitation beam of different wavelengths, in particular a laser device (4), in particular with a quantum cascade laser QCL, a tuneable QCL, and/or with a laser array, preferably an array of QCLs, for generating one or more excitation beams (10) of different wavelengths, preferably in the infrared or medium-infrared spectral range, wherein the excitation beam is directed at the substance (3) when the measuring body (1, 1a) is coupled and/or in contact with the substance (3) in the region of the measuring surface (2),
- a modulation device (8) for modulating the intensity of the excitation beam (10) at a modulation rate between 1 Hz and 10 kHz and
- a detection device (5, 6, 7) which is at least partially integrated into or connected to the measuring body (1, 1a), comprising the following:
• a source (5) for detection light, preferably coherent detection light (11), and
• a first optical waveguide structure (6), which can be or is connected to the detection light source and which guides the detection light, the refractive index of which, at least in some sections, is dependent on the temperature and/or pressure, the first optical waveguide structure having at least one section (9) in which the light intensity depends on a phase shift of detection light in at least one part of the optical waveguide structure (6) due to a change in temperature or pressure resulting from heat or pressure waves, which are caused by an absorption of the excitation beam in the substance (3), when the measuring body (1, 1a) is coupled with the substance (3) in the region of the measurement surface (2), and
• a measuring device (7) for the direct or indirect detection of the light intensity in said section (9) of the first optical waveguide structure (6), in which the light intensity depends on a phase shift of the detection light in at least one part of the first optical waveguide structure due to a change in temperature or pressure,
wherein said measuring body (1, 1a) is or comprises a substrate and said first waveguide structure (6) comprises at least one optical waveguide (15, 16) which is integrated in the substrate (1a).

2. Device according to Claim 1, **characterized in that** at least one section of a projection of the first optical waveguide structure (6) in the direction of the surface normal of the measuring surface (2) is superimposed with said measuring surface (2).

3. Device according to claim 1 or 2, comprising a housing (33) having a housing (33) which is designed as a portable housing, in particular as a housing which can be worn on the wrist of a person in the manner of a wristwatch, the excitation beam source (4) preferably being a laser device which is arranged outside the housing (33) and can be coupled for a measurement, the coupling being implemented in particular by means of a fibre-optic optical waveguide.

4. Device according to Claim 1, 2 or 3, **characterized in that** the detection device comprises an interferometric device, in particular an interferometer (12) and/or an optical waveguide resonance element, in particular a resonance ring (13) or a resonance plate.

5. Device according to any one of Claims 1 to 4, **characterized in that** the first optical waveguide structure (6), in particular an interferometric device of the first optical waveguide structure, comprises at least one fibre-optic optical waveguide (14), which is connected to the measuring body (1) at least in some sections.

6. Device according to any one of Claims 1 to 5, **characterized in that** the first optical waveguide structure (6) comprises at least one silicon optical waveguide, which is connected to an insulating substrate or is integrated into an insulating substrate, and in particular the silicon optical waveguide also being at least partially covered by an insulator, in particular SiO₂.

7. Device according to any one of the preceding claims, **characterized in that** the excitation beam (10), in particular in the region of the measuring surface of the measuring body or a region adjacent to the measuring surface (2), passes through the material of the measuring body (1, 1a) or a region adjacent to the measuring surface, wherein the measuring body or the region penetrated by the excitation beam (10) is transparent to the excitation beam.

8. Device according to any one of the preceding claims, **characterized in that** the excitation beam (10) is guided inside the measuring body (1, 1a) or along the measuring body by means of a second optical waveguide structure (17).

9. Device according to any one of the preceding claims, **characterized in that** the excitation beam (10) between the laser device (4) and the substance (3) to be analysed passes through a continuous opening (18) of the measuring body (1, 1a), wherein the opening ends in particular at a distance in front of the measuring surface or penetrates the measuring surface (2) or is arranged in a region which is directly adjacent to the measuring surface and/or adjoins it.

10. Device according to any one of the preceding claims, **characterized in that** the measuring body (1, 1a) is formed as a flat body, in particular as a plane-parallel body in the form of a plate, wherein in particular the thickness of the measuring body in the direction perpendicular to the measuring surface (2) is less than 50% of the smallest extension of the measuring body in a direction extending in the measuring surface, in particular, less than 25%, more particularly less than 10%.

11. Device according to any one of the preceding claims, **characterized in that** the measuring body (1, 1a) comprises or carries a mirror device (19) for reflecting the excitation beam (10) irradiated by the laser device (4) onto the measuring surface (2).

12. Device according to any one of the preceding claims, **characterized in that** the excitation beam (10) is oriented into the measuring body (1, 1a) parallel to the measuring surface (2) or at an angle of less than 30 degrees, in particular less than 20 degrees, more particularly less than 10 degrees or less than 5 degrees to the measuring surface, and that the excitation beam is diverted or deflected towards the measuring surface, wherein the excitation beam in particular passes through the measuring surface or an imaginary continuation of the measuring surface in the region of a continuous opening (18) in the measuring body.

13. Device according to any one of the preceding claims, **characterized in that** in the measuring body (1, 1a), behind and/or next to the detection device (5, 6, 7) viewed from the measuring surface (2), in particular behind and/or next to the first optical waveguide structure (6), in particular adjacent to and in thermal contact with the latter, at least one heat sink (20) is arranged in the form of a solid body or material, wherein in particular, the specific thermal capacity and/or specific thermal conductivity of the body or the material of the heat sink is greater than the specific thermal capacity and/or thermal conductivity of the material of the detection device (5, 6, 7) and/or of the first optical waveguide structure and/or the substrate (1a) of the first optical waveguide structure (6) and/or of the other materials which comprise the measuring body (1, 1a) and/or that a barrier (30, 40, 41) is provided in the measuring body (1, 1a), which at least partially shields a part of the detection device, in particular a part of the first optical waveguide structure (6), more particularly a reference arm of an interferometer, from the effect of the thermal and/or pressure wave and/or that the first optical waveguide structure (6) of the detection device comprises at least two measuring sections (15a, 16a), arranged in particular on different arms of an interferometer and in which the refractive index changes as a function of pressure and/or temperature changes, in particular of a pressure and/or thermal wave, so that a phase shift occurs in the detection light passing through the measuring sections followed by a resulting intensity change in the detection light in a further section as a function of pressure and/or temperature changes, the two measuring sections being arranged in the measuring body in such a way that they are passed through by a pressure and/or thermal wave, which propagates through the measuring body starting from the measuring surface (2), in particular from the region of the measuring surface in which the excitation beam penetrates it, one after the other, in particular in time intervals temporally shifted relative to one another or with a time delay.

14. Method for operating a device according to any one of the preceding claims, **characterized in that** a modulated excitation beam (10) is directed, in particular through the measuring body, onto the substance (3) to be analysed and that a temporal light intensity profile or waveform or a periodic light intensity change is detected by the detection device, these being detected for a plurality of wavelengths of the excitation beam by measuring the light intensity change in the first optical waveguide structure or by measuring the light intensity of light emitted from the first optical waveguide structure and obtaining an absorption spectrum of the substance to be analysed from the acquired data.

15. Method according to Claim 14, **characterized in that** the measurement is carried out for different modulation frequencies of the excitation beam (10) and that a corrected absorption spectrum is determined from the combination of absorption spectra obtained.

## Revendications

1. Dispositif pour l'analyse d'un matériau, avec :
- un corps de mesure (1, 1a) qui présente une surface de mesure (2) et qui doit être couplé, au niveau de la surface de mesure, pour la mesure, au moins partiellement, avec le matériau (3), plus particulièrement couplé directement ou à l'aide d'un milieu, plus particulièrement d'un fluide ou mis en contact directement ou également à l'aide d'un milieu, dans lequel la surface de mesure (2) présente la forme d'une surface plane ou présente une surface ou une partie de surface concave,
- une source de rayonnement d'excitation qui est capable de générer un rayonnement d'excitation de différentes longueurs d'ondes, plus particulièrement d'un dispositif laser (4), plus particulièrement un laser à cascade quantique QCL, un QCL accordable et/ou avec une matrice de laser, de préférence une matrice de QCL, pour la production d'un ou plusieurs rayonnements d'excitation (10) avec différentes longueurs d'ondes, de préférence dans le domaine infrarouge ou dans le domaine de l'infrarouge moyen, dans lequel le rayonnement d'excitation est dirigé vers le matériau (3) lorsque le corps de mesure (1, 1a) est couplé et/ou en contact avec le matériau (3) au niveau de la surface de mesure (2),
- un dispositif de modulation (8) pour la modulation de l'intensité du rayonnement d'excitation (10) avec une fréquence de modulation entre 1 Hz et 10 kHz et
- un dispositif de détection (5, 6, 7) intégré au moins partiellement dans le corps de mesure (1, 1a) ou relié avec celui-ci, qui comprend les éléments suivants :
• une source (5) de lumière de détection, de préférence une lumière de détection cohérente (11) et
• une première structure de guidage d'ondes lumineuses (6) reliée ou pouvant être reliée avec la source de lumière de détection et guidant la lumière de détection, dont l'indice de réfraction dépend, au moins à certains endroits, de la température et/ou de la pression, dans lequel la première structure de guidage d'ondes lumineuses présente au moins une portion (9) dans laquelle l'intensité lumineuse dépend d'un décalage de phase de la lumière de détection dans au moins une partie de la première structure de guidage d'ondes lumineuses (6), due à une variation de température ou de pression, suite à des ondes de chaleur et/ou de température, qui sont provoquées par l'absorption du rayonnement d'excitation dans le matériau (3), lorsque le corps de mesure (1, 1a) est couplé avec le matériau (3) au niveau de la surface de mesure (2) et
• un dispositif de mesure (7) pour le relevé direct ou indirect de l'intensité lumineuse dans la portion (9) mentionnée de la première structure de guidage d'ondes lumineuses (6), dans laquelle l'intensité lumineuse dépend du décalage de phase mentionné de la lumière de détection dans l'au moins une partie mentionnée de la première structure de guidage d'ondes lumineuses, dû à une variation de température ou de pression,
dans lequel le corps de mesure (1, 1a) constitue ou contient un substrat et la première structure de guidage d'ondes lumineuses (6) comprend au moins un guide d'ondes lumineuses (15, 16) qui est intégré dans le substrat (1, 1a).

2. Dispositif selon la revendication 1, **caractérisé en ce qu'**au moins une portion d'une projection de la première structure de guidage d'ondes lumineuses (6) est superposée, dans la direction de la normale à la surface d mesure (2), avec cette surface de mesure (2).

3. Dispositif selon la revendication 1 ou 2, avec un boîtier (33) qui est conçu comme un boîtier portatif, plus particulièrement comme un boîtier pouvant être porté au poignet d'une personne à la manière d'une montre-bracelet, dans lequel la source de rayonnement d'excitation (4) est de préférence un dispositif laser qui est disposé à l'extérieur du boîtier (33) et qui peut être couplé pour une mesure, dans lequel le couplage est réalisé plus particulièrement au moyen d'un guide d'ondes lumineuses à fibres optiques.

4. Dispositif selon la revendication 1, 2 ou 3, **caractérisé en ce que** le dispositif de détection comprend un dispositif interférométrique, plus particulièrement un interféromètre (12) et/ou un élément de résonance à guide d'ondes lumineuses, plus particulièrement un anneau de résonance (13) ou une plaque de résonance.

5. Dispositif selon l'une des revendications 1 à 4, **caractérisé en ce que** la première structure de guidage d'ondes lumineuses (6), plus particulièrement un dispositif interférométrique de la première structure de guidage d'ondes lumineuses, comprend un guide d'ondes lumineuses à fibres de verre (14), qui est relié fermement, au moins à certains endroits, avec le corps de mesure (1).

6. Dispositif selon l'une des revendications 1 à 5, **caractérisé en ce que** la première structure de guidage d'ondes lumineuses (6) comprend plus particulièrement au moins un guide d'ondes lumineuses en silicium ou est intégré dans un substrat isolant et dans lequel, en outre, plus particulièrement, le guide d'ondes lumineuses en silicium est recouvert au moins partiellement d'un isolant, plus particulièrement du SiO₂.

7. Dispositif selon l'une des revendications précédentes, **caractérisé en ce que** le rayonnement d'excitation (10) traverse le matériau du corps de mesure (1, 1a), plus particulièrement au niveau de la surface de mesure du corps de mesure ou une partie voisine de la surface de mesure (2) ou une directement adjacente à la surface de mesure, dans lequel le corps de mesure ou la partie traversée par le rayonnement d'excitation (10) est transparent pour le rayonnement d'excitation.

8. Dispositif selon l'une des revendications précédentes, **caractérisé en ce que** le rayonnement d'excitation (10) est guidé à l'intérieur du corps de mesure (1, 1a) ou est guidé au niveau du corps de mesure par une deuxième structure de guidage d'ondes lumineuses (17).

9. Dispositif selon l'une des revendications précédentes, **caractérisé en ce que** le rayonnement d'excitation (10) traverse, entre le dispositif laser (4) et le matériau à analyser (3), un évidement continu (18) du corps de mesure (1, 1a), dans lequel l'évidement se termine plus particulièrement à une certaine distance avant la surface de mesure ou traverse la surface de mesure (2) ou est disposé dans une zone qui est directement voisine de la surface de mesure et/ou qui est adjacente à celle-ci.

10. Dispositif selon l'une des revendications précédentes, **caractérisé en ce que** le corps de mesure (1, 1a) est conçu comme un corps plat, plus particulièrement comme un corps à faces planes et parallèles sous la forme d'une plaque, dans lequel, plus particulièrement l'épaisseur du corps de mesure représente, dans la direction perpendiculaire à la surface de mesure (2), moins de 50 % de l'extension la plus faible du corps de mesure dans une direction s'étendant dans la surface de mesure, plus particulièrement moins de 25 %, en outre plus particulièrement moins de 10 %.

11. Dispositif selon l'une des revendications précédentes, **caractérisé en ce que** le corps de mesure (1, 1a) comprend ou supporte un dispositif de miroir (19) pour la réflexion du rayonnement d'excitation (10) émis par le dispositif laser (4) vers la surface de mesure (2).

12. Dispositif selon l'une des revendications précédentes, **caractérisé en ce que** le rayonnement d'excitation (10) est orienté parallèlement à la surface de mesure (2) ou avec un angle inférieur à 30 degrés, plus particulièrement inférieur à 20 degrés, plus particulièrement inférieur à 10 degrés ou inférieur à 5 degrés par rapport à la surface de mesure, vers le corps de mesure (1, 1a), et **en ce que** le rayonnement d'excitation est renvoyé ou dévié en direction de la surface de mesure, dans lequel le rayonnement d'excitation traverse plus particulièrement la surface de mesure ou un prolongement imaginaire de la surface de mesure au niveau d'un évidement continu (18) dans le corps de mesure.

13. Dispositif selon l'une des revendications précédentes, **caractérisé en ce que**, dans le corps de mesure (1, 1a), vu à partir de la surface de mesure (2), derrière et/ou près du dispositif de détection (5, 6, 7), plus particulièrement de la première structure de guidage d'ondes lumineuses (6), plus particulièrement de manière adjacente à celle-ci et en contact thermique avec celle-ci, se trouve au moins un dissipateur thermique (20) sous la forme d'un corps ou d'un matériau solide, dans lequel, plus particulièrement, la capacité thermique spécifique et/ou la conductivité thermique spécifique du corps ou du matériau du dissipateur thermique est supérieure à la capacité thermique spécifique et/ou la conductivité thermique spécifique du matériau du dispositif de détection (5, 6, 7) et/ou de la première structure de guidage d'ondes lumineuses et/ou du substrat (1a) de la première structure de guidage d'ondes lumineuses (6) et/ou des autres matériaux dont est constitué le corps de mesure (1, 1a) et/ou **en ce que**, dans le corps de mesure (1, 1a), est prévue une barrière (30, 40, 41) qui éloigne au moins partiellement l'influence de l'onde de chaleur et/ou de pression d'une partie du dispositif de détection, plus particulièrement d'une partie de la première structure de guidage d'ondes lumineuses (6), en outre plus particulièrement d'un bras de référence d'un interféromètre et/ou **en ce que** la première structure de guidage d'ondes lumineuses (6) du dispositif de détection comprend au moins deux portions de mesure (15a, 16a) qui sont disposées plus particulièrement sur des bras différents d'un interféromètre et dans lesquelles l'indice de réfraction varie en fonction des variations de pression et/ou de température, plus particulièrement d'une onde de pression et/ou de chaleur, de sorte qu'un décalage de phase de la lumière de détection traversant les portions de mesure et une variation de l'intensité de la lumière de détection, qui en résulte, a lieu une autre portion en fonction des variations de pression et/ou de température, dans lequel les deux portions de mesure sont disposées dans le corps de mesure de sorte qu'elles soient traversées, par une onde de pression et/ou de chaleur, qui se propage à partir de la surface de mesure (2), plus particulièrement de la partie de la surface de mesure dans laquelle le rayonnement d'excitation la traverse, à travers le corps de mesure, successivement dans le temps, plus particulièrement pendant des périodes décalées entre elles ou avec un intervalle de temps.

14. Procédé de fonctionnement d'un dispositif selon l'une des revendications précédentes, **caractérisé en ce qu'**un rayonnement d'excitation modulé (10) est dirigé, plus particulièrement à travers le corps de mesure, vers le matériau à analyser (3) et **en ce que** le dispositif de détection relève un tracé de l'intensité lumineuse ou une variation périodique de l'intensité lumineuse, dans lequel celle-ci est relevée pour une pluralité de longueurs d'ondes du rayonnement d'excitation par la mesure de la variation d'intensité lumineuse dans la première structure de guidage d'ondes lumineuses ou par la mesure de l'intensité lumineuse de la lumière sortant de la première structure de guidage d'ondes lumineuses et, à partir des données relevées, un spectre d'absorption du matériau à analyser est obtenu.

15. Procédé selon la revendication 14, **caractérisé en ce que** la mesure est effectuée pour différentes fréquences de modulation du rayonnement d'excitation (10) et **en ce que**, à partir de la combinaison des spectres d'absorption obtenus, un spectre d'absorption corrigé est déterminé.
